# EUROPEAN PATENT APPLICATION

(11) **EP 3 208 328 A1**
(43) Date of publication of application: **23.08.2017**
(21) Application number: 15850423.3
(22) Date of filing: 16.10.2015
(51) Int. Cl.: C12N 5/077, A61K 35/12, A61P 9/00, C12M 1/34, C12M 3/00, C12N 5/0735

(54) **TISSUE FRAGMENT**

(30) Priority: 16.10.2014 JP 2014212008; 16.10.2014 JP 2014212010; 16.10.2014 JP 2014212014
(71) Applicant: Kyoto University, Kyoto-shi, Kyoto 606-8501 (JP)
(72) Inventor: LIU, Li, Kyoto-shi Kyoto 606-8501 (JP); LI, Junjun, Kyoto-shi Kyoto 606-8501 (JP); MINAMI, Itsunari, Kyoto-shi Kyoto 606-8501 (JP); CHEN, Yong, Kyoto-shi Kyoto 606-8501 (JP); NAKATSUJI, Norio, Kyoto-shi Kyoto 606-8501 (JP)
(74) Representative: dompatent von Kreisler Selting Werner - Partnerschaft von Patent- und Rechtsanwälten mbB
(86) International application number: PCT/JP2015/079364
(87) International publication number: WO 2016/060260

(57) **Abstract**

Provided is a method for producing a tissue-like construct having cultured cells organized in an aligned manner, especially, a cardiac tissue-like construct. The tissue-like construct prepared by this method is also provided. Further provided are a device for evaluating cellular electrophysiological functions and a method for evaluating cellular electrophysiological functions. Furthermore, a sheet-like cell culture scaffold is also provided.

## Description

### ART RELATED

The present invention relates to a method for producing a tissue-like construct comprising cultured cells aligned in one direction. The application also provides the tissue-like construct produced by the present method.

The invention further provides a device for evaluating the electrophysiological functions of cells, and a method for evaluating the electrophysiological functions of cells.

### BACKGROUND ART

There are approximately 800,000 people suffering from serious heart failure including myocardial infarction in Japan, and about 180,000 people died annually. At present, heart transplantation is the only effective treatment, but in Japan there is extremely serious donor shortage and transplantation has problems such as immunological rejection. Pluripotent stem cells such as embryonic pluripotent stem (ES) cells and induced pluripotent stem (iPS) cells have become available. It has been expected to restore myocardial function by cardiomyocyte regeneration therapy as an alternative to the heart transplantation. For clinical use in transplantation therapy, it is necessary to obtain a highly mature and safe cardiac tissue-like construct having the anisotropic structure similar to the cardiac tissue in the living body.

Various procedures for inducing cardiomyocytes from human ES/iPS cells have been proposed. The common procedures currently used to induce cardiomyocytes include a suspension culture of the ES/iPS cells in the presence of embryoid body-relating cytokines such as DKKl, bFGF, activin A and BMP4 as well as a proximal an adhesive co-culture of the ES/iPS cells with mouse visceral endoderm like(END2) cells (Non-Patent Literature 1 to 4).

The inventors had proposed a new method for inducing clinical-grade cardiomyocytes efficiently by using a small molecule to give a cell culture comprising up to about 98% of cardiomyocytes (Non-patent literature 5 and Patent literature 1). The cardiomyocytes prepared by the method express a relatively low degree of β-MHC, an important maturity marker of cardiomyocytes, as low as about 10% of the expression level of the normal adult human cardiomyocytes. In addition, the cells did not form the anisotropic structure and were not deemed matured based on the electrophysiological analysis.

There are two proposed methods in principle for cardiomyocytes transplantation: (1) the cells are directly injected into the disease area in the patient of heart failure, and (2) cardiomyocytes are formed into a sheet like construct and the construct is implanted to the disease area.

Non-Patent Literature 3 reports that cardiac infarction in guinea pigs had successfully been improved by infusing cardiomyocytes derived from iPS cells directly into the animals (Non patent literature 6). However, the direct injection of the cardiomyocytes may cause leakage of the injected cells. The leaked cells could disperse in the whole body and therefore, the low efficiency of the implantation therapy has been criticized. In addition, the cell engraft rate has been not more than several per cents. The therapeutic effect is not believed to be sufficient.

Method for creating cell sheets for implantation using temperature-responsive dishes has been proposed (Non-Patent Literature 7). Clinical studies in which implanting cardiomyocyte sheets that are created by this technique have been conducted. However, the cardiomyocyte sheets created by this technique are monolayer of non-anisotropic randomly arranged cells and do not mimic the in vivo structure of cardiomyocytes. Therefore, the sheet has problems of weak cardiac contraction force, low cellular maturity and the strength of the sheets are not enough for handling. In addition, there is no report of cardiomyocyte sheet that can be implanted with the substrate on which the cell sheet was created.

Artificial cell sheets that mimic the in vivo cellular structure are desired to be created for use in efficient implantation or assay. Researches to imitate the cellular structure in the living body with microengineering techniques have been attracting attention. For example, in adult myocardium, cardiomyocytes are longitudinally aligned in the form of parallel bundles. In order to mimic this nature's work, engineered anisotropy of the cardiomyocytes has been widely tried by means of microline, micropunch and fibers (Non Patent Literature 8-14).

Primary culture of cells derived from an animal and animals have been used to evaluate safety of a substance in the development of pharmaceutical products. However, they have various problems such as differences in the functions from the cells in the human living body due to the differences in species and lots. At present, according to the guidance issued by the Government, non-clinical data regarding cardiotoxicity of the product evaluated by QT interval prolongation of electrocardiogram is required for developing a new drug product in Japan. There is high demand in the pharmaceutical industry for inducing cardiomyocytes from pluripotent stem cells such as iPS cells and producing a cardiac tissue-like construct by culturing the cardiomyocytes that can be used for evaluation of toxicity and kinetics of drugs.

The patch clamp method which can record the electric potential in a cell is used generally for the electrophysiological evaluation of the cell. In the patch clamp method, the cell is insulted by the patch electrode. The method is invasive and the long-term measurement is difficult. In addition, the method is not suitable for screening because the operation of the method is difficult.

Multi-electrode array (MEA) systems have been making a remarkable development. Nowadays, it has become possible to incubate cardiomyocytes induced from human ES/iPS cells on a MEA and to evaluate their electrophysiological properties. (Non-patent literatures 15 and 16). According to this technique, extracellular electric activities are determined and therefore, this method does not damage the cells and can be used to observe and evaluate the cells for long term while culturing the cells. With this technique, it becomes possible not only to evaluate the toxicity of a drug candidate indispensable for drug discovery screening but also to monitor differentiation and maturation process from pluripotent stem cells such as human ES cells and iPS cells to cardiomyocytes.

In cardiac tissue constructs obtained by conventional procedures for differentiating pluripotent stem cells into cardiomyocyte, the cells are randomly-oriented and do not form an anisotropic alignment. There are still problems including weak muscle contracting power and frequent occurrence of arrhythmia when an electrocardiogram is obtained such as for determining QT interval prolongation. The cells are weakly adhered to the electrodes and therefore, this system is not suitable for long-term incubation. Accordingly the conventional system cannot be used as a stable system or drug discovery screening. It is hard to say that the electrical signal from cardiac tissue in which cardiomyocytes proliferate in a randomly-oriented manner or in a bulk shape sufficiently imitates the in vivo behavior of the myocardial tissues.

### PRIOR ART REFERENCES

### Patent Literature

Patent Literature 1: WO2013/111875

### Non Patent Literature

Non Patent Literature 1: Laflamme,M.A. & Murry, C.E. Heart regeneration. Nature 473,326-335(2011).
Non Patent Literature 2: Rajala, K., Pekkanen-Mattila, M. & Aalto-Setala,K. Cardiac differentiation of pluripotent stem cells. Stem Cells Int 2011,383709(2011).
Non Patent Literature 3: Yang,L. et al. Human cardiovascular progenitor cells develop from a KDR+ embryonic-stem-cell-derived population. Nature 453, 524-528 (2008).
Non Patent Literature 4: Paige,S.L. et al. Endogenous Wnt/beta-catenin signaling is required for cardiac differentiation in human embryonic stem cells. PLoS One 5,e11134(2010).
Non Patent Literature 5: Minami I, et al. A small molecule that promotes cardiac differentiation of human pluripotent stem cells under defined, cytokine- and xeno-free conditions. Cell Rep. 2012 Nov 29; 2(5): 1448-60.
Non Patent Literature 6: Shiba Y,et al. Human ES-cell-derived cardiomyocytes electrically couple and suppress arrhythmias in injured hearts. Nature 489, 322-325 (2012)
Non Patent Literature 7: Kawamura M,et al. Enhanced survival of transplanted human induced pluripotent stem cell-derived cardiomyocytes by the combination of cell sheets with the pedicled omental flap technique in a porcine heart. Circulation,128,587-594(2013)
Non Patent Literature 8: D.-H. Kim et al. "Nanoscale cues regulate the structure and function of macroscopic cardiac tissue-like constructs" PNAS. 12(2),565-570(2010)
Non Patent Literature 9: D.-H. Kim et al. "Nanopatterned cardiac cell patches promote stem cell niche formation and myocardial regeneration" Integrative Biology 4,1019-1033(2012)
Non Patent Literature 10: Donghui Zhang et al. "Tissue-engineered cardiac patch for advanced functional maturation of human ESC-derived cardiomyocytes" 34 5813-5820 (2013)
Non Patent Literature 11: C.-W Hsiao et al. "Electrical coupling of isolated cardiomyocyte clusters grown on aligned conductive nanofibrous meshes for their synchronized beating" Biomaterials 34,1063-1072(2013)
Non Patent Literature 12: X. Zong et al. "Electrospun fine-textured scaffolds for heart tissue-like constructs" Biomaterials 26,5330-5338(2005)
Non Patent Literature 13: I. C. Parrag et al. "Fiber alignment and coculture with fibroblasts improves the differentiated phenotype of murine embryonic stem cell-derived cardiomyocytes for cardiac tissue engineering" 109 (39), 813-822 (2012)
Non Patent Literature 14: J. Wang et al. "Effect of engineered anisotropy on the susceptibility of human pluripotent stem cell-derived ventricular cardiomyocytes to arrhythmias" Biomaterials 34(35),8878-8886(2013)
Non Patent Literature 15: Otsuji T. G. et al. Progressive maturation in contracting cardiomyocytes derived from human embryonic stem cells: Qualitative effects on electrophysiological responses to drugs. Stem Cell Research,4,201-213(2010)
Non Patent Literature 16: A. L. Lahti,et al. Silvennoinen and K. Aalto-Setala "Model for long QT syndrome type 2 using human iPS cells demonstrates arrhythmogenic characteristics in cell culture. Sisease Models & Mechanisms,5,220-230(2010)

### SUMMARY OF THE INVENTION

### PROBLEMS TO BE SOLVED BY THE INVENTION

In the first aspect, an object of the present invention is to provide a method for manufacturing a tissue-like construct having cells aligned in one direction, for example, a cardiac tissue-like construct. Another object of the present invention under the first aspect is to provide a tissue-like construct that can be generated by the method and has cultured cells aligned in one direction, and method for evaluating the tissue-like construct.

In the second aspect, an object of the present invention is to provide a device for evaluating the electrophysiological function of cultured cells.

In the third aspect, an object of the present invention is to provide a sheet-like cell culture scaffold that can be used as cell culture scaffold.

### MEANS TO SOLVE THE PROBLEMS

The present invention provides the followings:
(1) A cardiac tissue-like construct comprising aligned fiber sheet and cultured cardiomyocytes on the sheet.
(2) The cardiac tissue-like construct according to (1), wherein the aligned fiber sheet is between the cultured cardiomyocyte layers.
(3) The cardiac tissue-like construct according to (1) or (2), wherein the cultured cardiomyocytes are those induced from pluripotent stem cells.
(4) The cardiac tissue-like construct according to any one of (1) to (3), wherein the diameter of the fibers constituting the aligned fiber sheet is 0.1 µm-5µm, the number of the fibers within 1mm width of the sheet is 30-15000, and the thickness of the sheet is 0.1 µm-20µm.
(5) The cardiac tissue-like construct according to any one of (1) to (4), which comprises 10 or more cardiomyocyte layers.
(6) The cardiac tissue-like construct according to any one of (1) to (5), wherein the fibers constituting the aligned fiber sheet is made from a biodegradable material.
(7) The cardiac tissue-like construct according to any one of (1) to (5), wherein the fibers constituting the aligned fiber sheet is made from a hardly degradable material.
(8) The cardiac tissue-like construct according to any one of (1) to (7), wherein the aligned fiber sheet has a frame around the sheet.
(9) The cardiac tissue-like construct according to (8), wherein the frame is made from the same material as the aligned fiber sheet.
(10) The cardiac tissue-like construct according to (8), wherein the frame is made from a material other than the aligned fiber sheet.
(11) The cardiac tissue-like construct according to any one of (1)-(10), which comprises a plurality of aligned fiber sheets and a plurality of cardiomyocyte layers cultured on the fiber sheets, and the aligned fiber sheets are interposed between cardiomyocyte layers so as to the fiber direction of the aligned fibers are same.
(12) The cardiac tissue-like construct according to any one of (1)-(4), (6) and (7), further comprises a cell culture chamber on the aligned fiber sheet, and the cardiomyocytes are in the culture chamber.
(13) The cardiac tissue-like construct according to any one of (1)-(12), wherein the expression level of β-MHC in the cardiomyocytes in the cardiac tissue-like construct is 10% or more of the normal cardiomyocytes in adult human.
(14) The cardiac tissue-like construct according to any one of (1)-(13), which is for use in evaluating cell functions.
(15) The cardiac tissue-like construct according to any one of (6), (8) - (11) and (13), which is for use in implantation.
(16) A method for producing the cardiac tissue-like construct of (11), which comprises the step of stacking a plurality of cardiac tissue-like constructs so that the aligned fibers in each construct are in the same direction and culturing the stacked constructs.
(17) The method according to (16), further comprising the step of evaluating the cardiac tissue-like construct based on electrical signals from the cultured cardiomyocytes detected by means of a multiple electrode array that is contacted with the cardiac tissue-like construct.
(18) A method for evaluating the function of cardiomyocytes, which comprises the steps of contacting the cardiac tissue-like construct according to (14) with a multiple electrode array and detecting the electrical signals from the cardiac tissue-like construct.
(19) The method according to (18), which is for evaluating the effectiveness of a drug candidate substance based on the function of cardiomyocytes.
(20) The method according to (18), which is for evaluating the safety of a substance based on the function of cardiomyocytes.
(21) A product comprising a sterilized package and a cardiac tissue-like construct according to any one of (1)-(15) enclosed in the package.
(22) A device for evaluating function of cardiomyocytes, comprising, a multiple electrode array, and an aligned fiber sheet on the multiple electrode array.
(23) The device according to (22), further comprises a cell culture chamber moiety on the aligned fiber sheet.
(24) The device according to (23), further comprises cultured cardiomyocytes in the chamber moiety.
(25) A method for evaluating cardiomyocytes function, which comprises the step of detecting the electrical signal from the cultured cardiomyocytes in the device of (24).
(26) A sheet-shaped cell culture scaffold, comprising a sheet-shaped cell culture area and a frame provided around the cell culture area.
(27) The sheet-shaped cell culture scaffold according to (26), wherein the sheet-shaped cell culture area is made of an aligned fiber sheet.
(28) The sheet-shaped cell culture scaffold according to (26) or (27), wherein the sheet-shaped cell culture area and the frame are made from same material.
(29) The sheet-shaped cell culture scaffold according to (26) or (27), wherein the sheet-shaped cell culture area and the frame are made from different materials.
(30) The sheet-shaped cell culture scaffold according to any one of (27)-(29), wherein the sheet-shaped cell culture area is made of a randomly integrated fiber sheet.
(31) The sheet-shaped cell culture scaffold according to any one of (27)-(29), wherein the sheet-shaped cell culture area is made of an aligned fiber sheet.
(32) The sheet-shaped cell culture scaffold according to any one of (27)-(31), wherein the sheet-shaped cell culture area is made from a biodegradable material.
(33) The sheet-shaped cell culture scaffold according to any one of (27)-(31), wherein the sheet-shaped cell culture area is made from a hardly degradable material.
(34) The sheet-shaped cell culture scaffold according to any one of (26)-(33), which has a spacer on the frame.
(35) A tissue-like construct comprising: the sheet-shaped cell culture scaffold according to any one of (26)-(34) and cultured cells on the scaffold.
(36) The tissue-like construct according to (35), wherein a plurality of sheet-shaped cell culture scaffolds are interposed between cultured cell layers.
(37) A product comprising, a sterilized package, and a tissue-like construct according to (35) or (36) enclosed in the package.

### EFFECT OF THE INVENTION

According to the present invention, a tissue-like construct having culture cells aligned in one direction can be obtained. The tissue-like construct having cells aligned in one direction is obtained by culturing cells that are aligned in one direction in the living body such as cardiomyocytes by the method of the present invention. The cells in the cardiac tissue-like construct are highly matured compared with randomly cultured cells and hardly occur arrhythmia. The tissue-like construct is preferably used for implantation and drug screening.

[Figure 1] A schematic diagram explaining embodiments of the preparation of cardiac tissue-like construct on the aligned fiber sheet, and of the evaluation of the function of cardiomyocytes with a MEA coated with the aligned fiber sheet.
[Figure 2A] Electron microscopic images of PLGA electrospun aligned fibers (AF) and random fibers (RA).
[Figure 2B] Distributions of diameters of fibers constituting the PLGA aligned fiber sheet (AF) and PLGA random fiber sheet (RF).
[Figure 2C] Resiliency of PLGA aligned fiber sheet (AF) and PLGA random fiber sheet (RF).
[Figure 2D] Change of diameters of electrospun PLGA fibers depending on the concentration of the polymer solution.
[Figure 2E] Angular distributions of PLGA aligned fibers electrospun for 40 seconds.
[Figure 2F] Microscopic images of PLAG aligned fibers electrospun for 90 seconds (left) and 300 seconds (right). The scale bar represents 50 µm
[Figure 2G] Angular distributions of PLGA aligned fibers electrospun for 90 seconds.
[Figure 2H] Thickness of each PMGI fiber sheet shown in Figure 2F.
[Figure 2I] Number of fibers per 1 mm thickness of each PMGI fiber sheet shown in Figure 2F.
[Figure 3A] Cardiomyocytes attachment rate when the cells seeded on the PLGA aligned fiber sheet-coated substrates (AF), PLGA random fiber sheet-coated substrates (RA), gelatin-coated flat substrates or PLGA polymer-coated flat substrates.
[Figure 3B] PLGA aligned fiber sheets obtained by electrospining for 10 seconds, 40 seconds, 10 minutes and 15 minutes respectively.
[Figure 3C] Thickness of each fiber sheet obtained by the conditions shown in Figure 3B.
[Figure 3D] Number of fibers per 1 mm in width of each fiber sheet obtained by the condition shown in Figure 3B.
[Figure 3E] Cell attachment rate on each fiber sheet obtained by the condition shown in Figure 3B.
[Figure 3F] Attachment rates of cardiomyocytes induced from human iPS cells (IMR90-1) on high- and low-density PMGI aligned fiber sheet-coated substrates and gelatin-coated substrates.
[Figure 4A] A photograph of a device having electrodes, an aligned fiber sheet and a PDMS cell culture chamber, wherein the electrode is coated with the aligned fiber sheet and the chamber is placed on the fiber sheet.
[Figure 4B] Photographs of devices having a MEA coated with a PS aligned fiber sheet and a MEA coated with a PLGA aligned fiber sheet.
[Figure 5A] Photographs of cardiomyocyte culture, the cells were cultured for 2 days on a PMGI aligned fiber sheet-coated (AF) MEA (Left) and on a gelatin-coated (Flat) MEA (Right).
[Figure 5B] Photographs of cardiomyocyte culture, the cells were cultured for 14 days on the PMGI aligned fiber sheet-coated (AF) MEA (left), the aligned fiber sheet with cultured cells detached from the MEA (upper right) and the surface of the MEA from which the aligned fiber sheet on which the cardiomyocytes were cultured was peeled off.
[Figure 5C] Time course of electrical signals detected when cardiomyocytes were cultured for 14 days on the PMGI aligned fiber sheet-coated MEA (AF) and on the gelatin coated MEA (Flat).

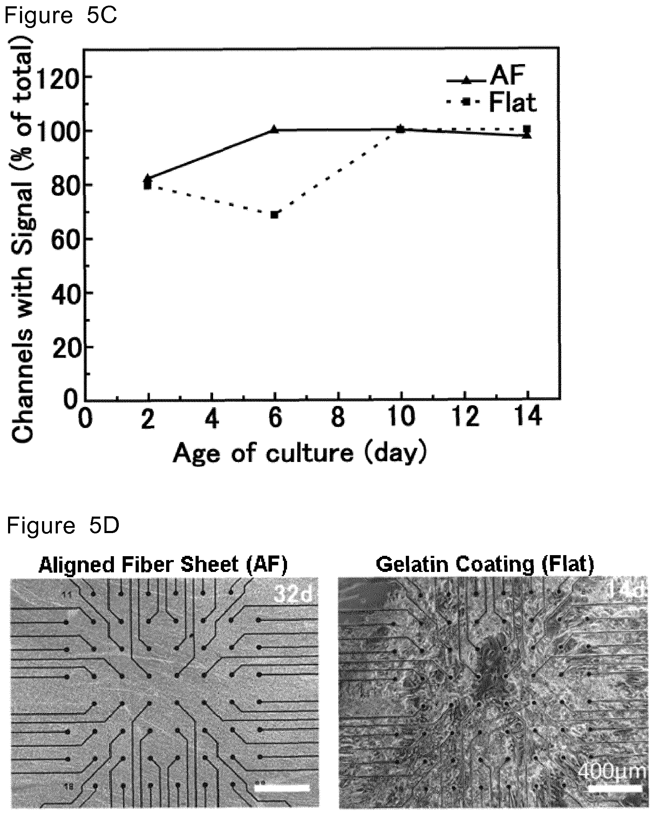

[Figure 5C] Photographs of cardiomyocytes cultured on the PMGI aligned fiber sheet-coated MEA (AF) for 32 days and on the gelatin coated MEA (Flat) for 14 days.

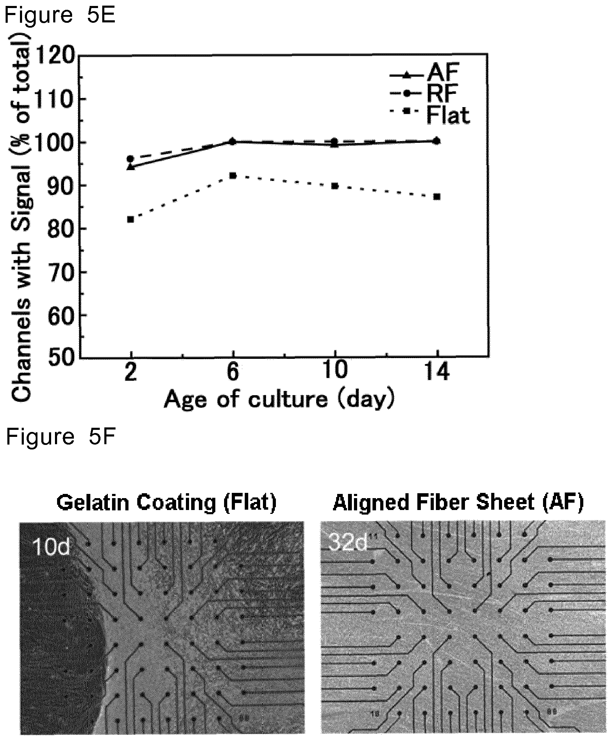

[Figure 5E] Time course of electrical signal detected rates when cardiomyocytes were cultured for 14 days on a PLGA aligned fiber sheet-coated MEA (AF), on a PLGA random fiber sheet-coated MEA (RF) and on a gelatin coated MEA (Flat).
[Figure 5F] Photographs of cardiomyocytes cultured on the gelatin coated MEA (Flat) for 10 days (left) and on the PLGA aligned fiber sheet-coated MEA (AF) for 32 days (right).

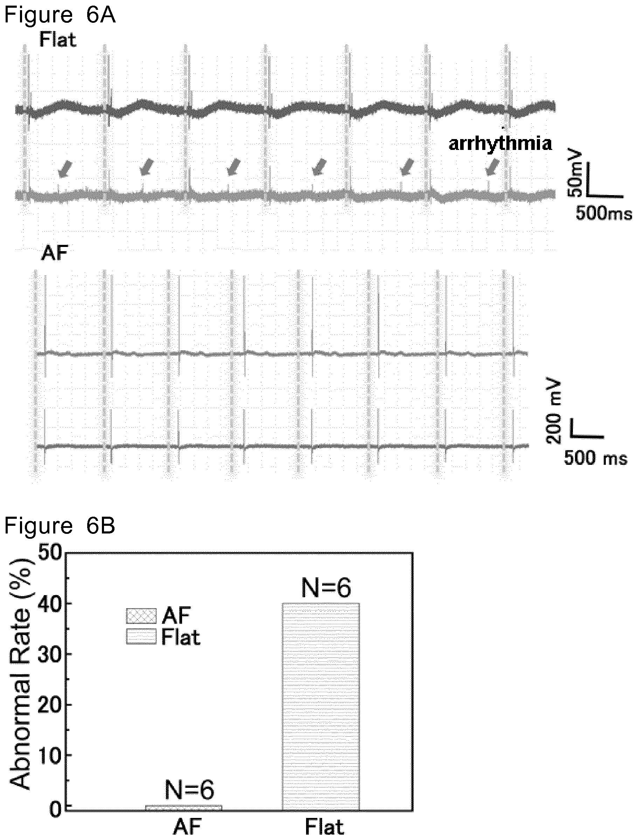

[Figure 6A] Electrical activity patterns of cardiomyocytes cultured for 14 days on a PMGI aligned fiber sheet-coated MEA (AF) and on a gelatin-coated MEA (Flat).
[Figure 6B] Frequency of arrhythmia detected in the electrical activity patterns of Figure 6A.

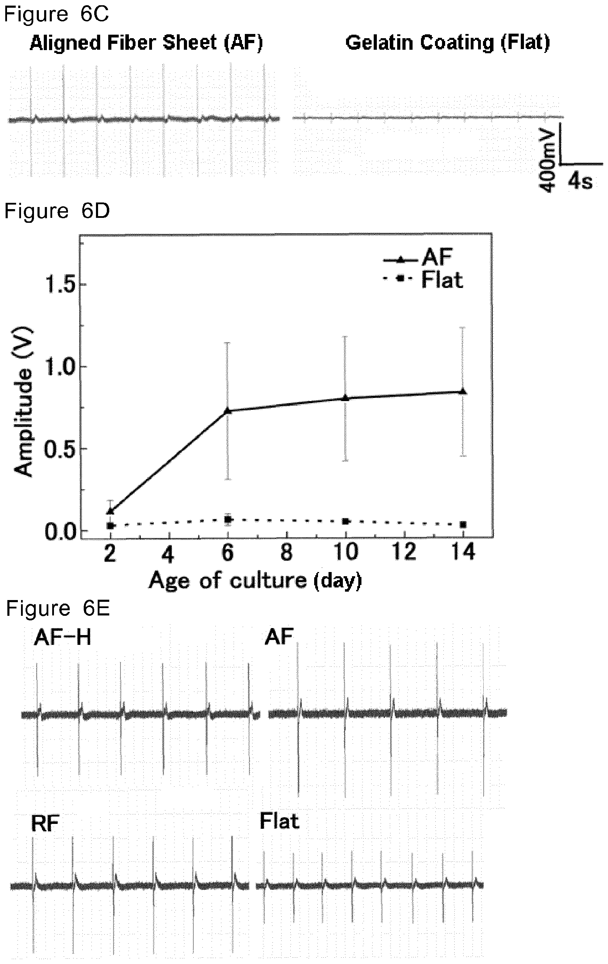

[Figure 6C] Amplitudes of the electrical activities of the cardiomyocytes cultured for 6 days on the PMGI aligned fiber sheet-coated MEA(AF)and on the gelatin-coated MEA(Flat).
[Figure 6D] Time course of amplitudes of the electrical activities of the cardiomyocytes cultured for 14 days on each of the MEAs shown in Figure 6A.
[Figure 6E] Electrical activity patterns of cardiomyocytes cultured on the high density PLGA aligned fiber sheet-coated (AF-H), low density PLGA aligned fiber sheet-coated (AF), random PLGA fiber sheet-coated (RF) and gelatin-coated (Flat) MEAs, respectively.

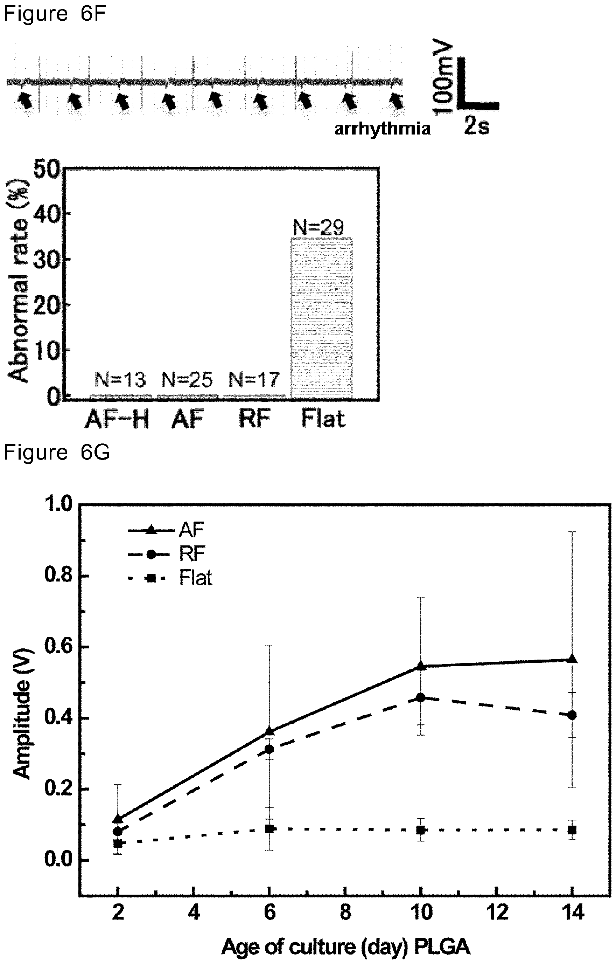

[Figure 6F] Arrhythmias observed in cardiomyocytes cultured on the gelatin-coated MEA (upper) and frequency of arrhythmias detected in cardiomyocytes cultured on the high density PLGA aligned fiber sheet-coated (AF-H), low density PLGA aligned fiber sheet-coated (AF), random PLGA fiber sheet-coated (RF) and gelatin-coated (Flat) MEAs (lower).
[Figure 6G] Time course of signal amplitudes in the cardiomyocytes cultured on the PLGA aligned fiber sheet-coated (AF), random PLGA fiber sheet-coated (RF) and gelatin-coated (Flat) MEAs.

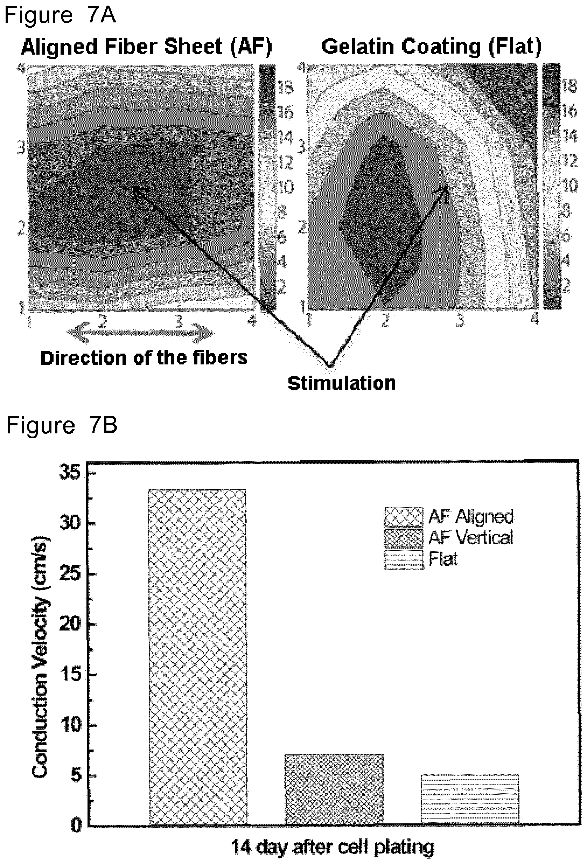

[Figure 7A] Activation maps showing the propagation of the excitation observed when the cardiomyocytes that were cultured on the PMGI aligned fiber sheet-coated (AF) MEA and gelatin-coated (Flat) MEA for 14 days were given a single electrical stimulation.
[Figure 7B] The propagation speeds of the cell excitation in the cardiomyocytes cultured on the devices shown in Figure 7A at day 14 of culture.

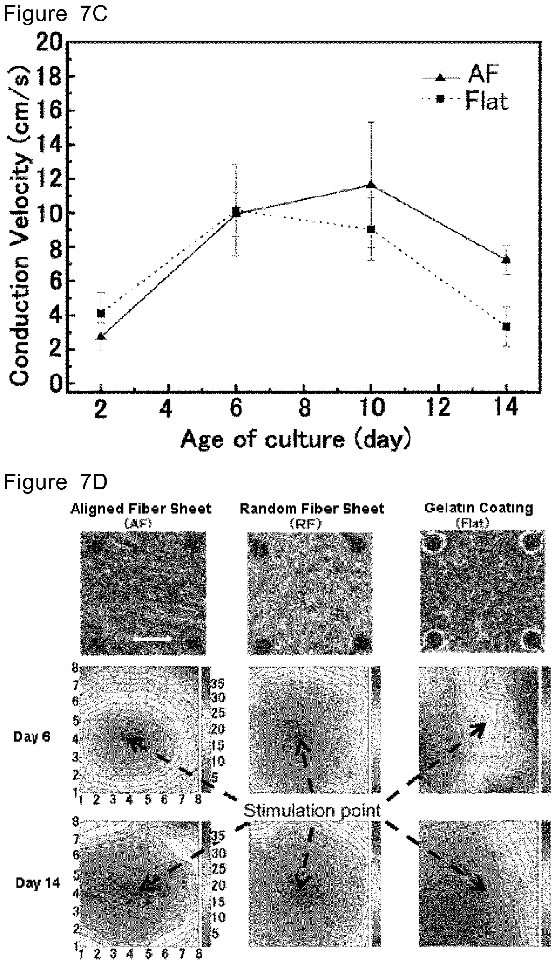

[Figure 7C] The propagation speeds of the cell excitation in the cardiomyocytes cultured on the respective devices shown in Figure 7A at different culture times from day 2 to day 14.
[Figure 7D] Activation maps showing the propagation of the excitation observed when the cardiomyocytes that were cultured on the PLGA aligned fiber sheet-coated (AF), random fiber sheet-coated (RF) and gelatin-coated (Flat) MEAs were given a single electrical stimulation at day 6 and day 14 of culture.

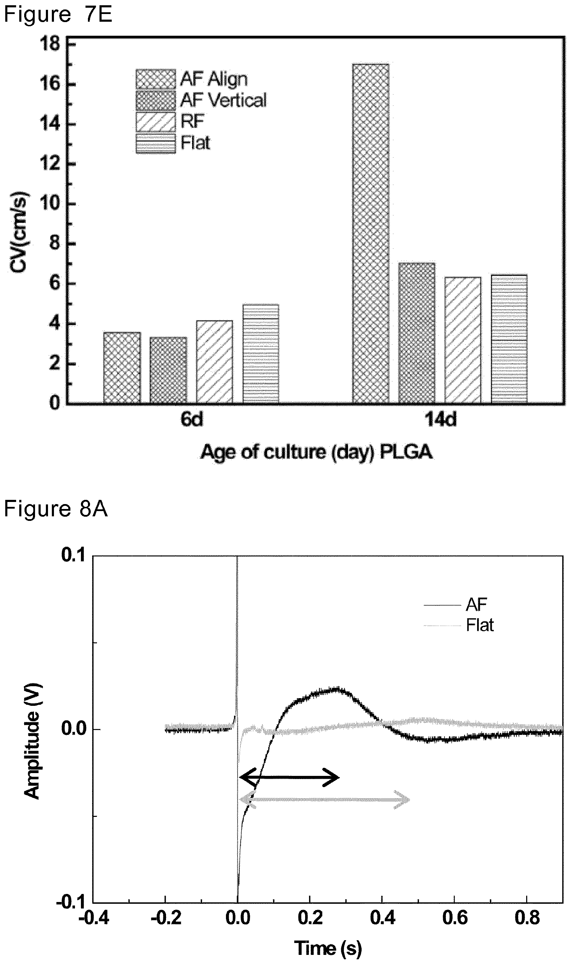

[Figure 7E] The propagation speeds of the cell excitation in the cardiomyocytes that were cultured on the PLGA aligned fiber sheet-coated (AF), random fiber sheet-coated (RF) and gelatin-coated (Flat) MEAs were given single electrical stimulation at day 6 and day 14 of culture.
[Figure 8A] QT intervals of the cardiomyocytes that were cultured on the PMGI aligned fiber sheet-coated (AF) and gelatin-coated (Flat) MEAs for 6 days.

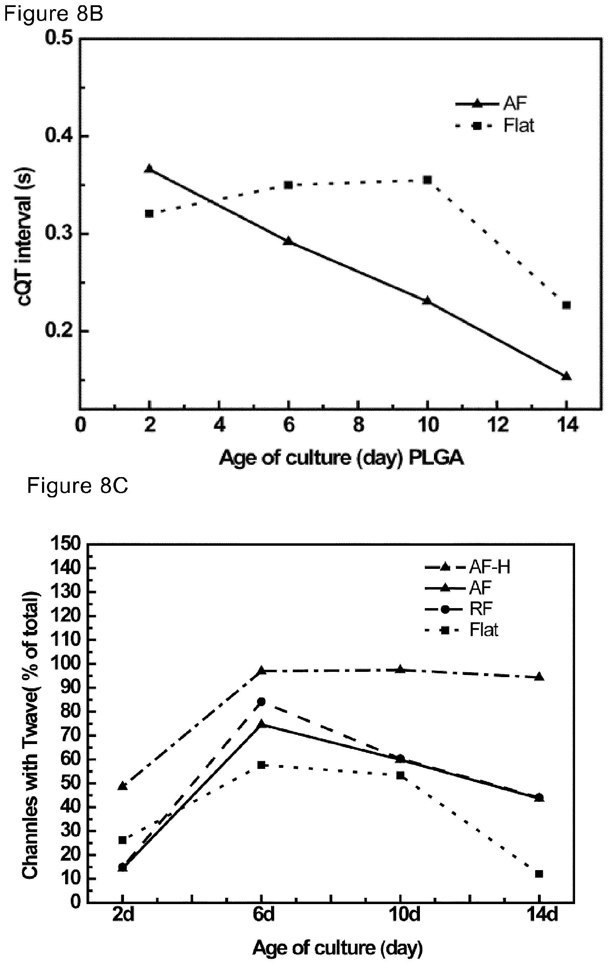

[Figure 8B] QT intervals of the cardiomyocytes cultured on the MEAs of Figure 8A measured at different culture periods, from day 2 to day 14.
[Figure 8C] The ratio of the channels recording T-wave of the cardiomyocytes that were cultured on the PLGA aligned fiber sheet-coated (AF :40 s spin time and AF-H: high density, 10 min spin time), random fiber sheet-coated (RF) and gelatin-coated (Flat) MEAs at different culture period.

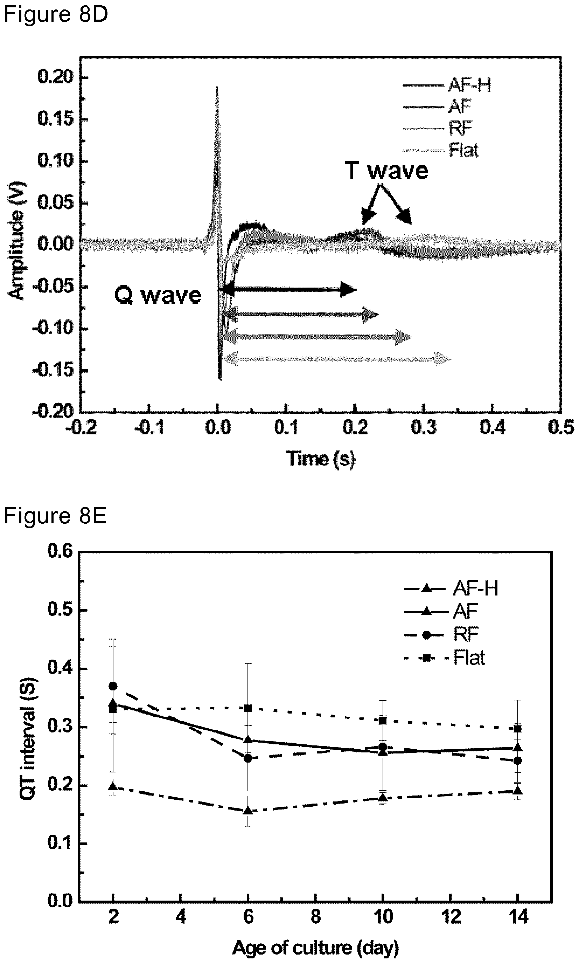

[Figure 8D] QT interval prolongation observed with the cardiomyocytes that were cultured on the PLGA aligned fiber sheet-coated (AF :40 s spin time and AF-H: high density, 10 min spin time), random fiber sheet-coated (RF) and gelatin-coated (Flat) MEAs at day 10 of culture.
[Figure 8E] QT intervals of the cardiomyocytes that were cultured on the PLGA aligned fiber sheet-coated (AF :40 s spin time and AF-H: high density, 10 min spin time), random fiber sheet-coated (RF) and gelatin-coated (Flat) MEAs at different culture period.

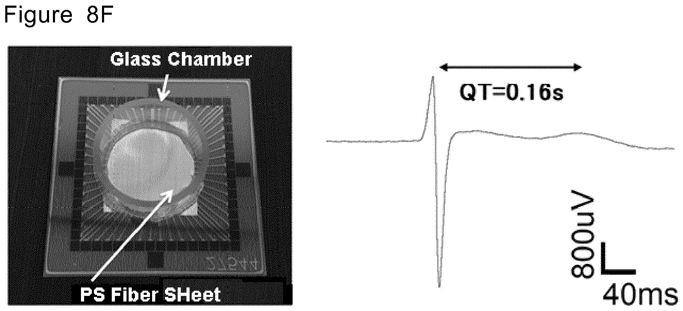

[Figure 8F] A device for evaluating cultured cell functions having a MEA, a PS aligned fiber sheet and a glass chamber, wherein the PS aligned fiber sheet is attached on the MEA and the glass chamber is provided on the PS aligned fiber sheet (left). QT interval evaluated on the device at day 14 of culture of the cardiomyocytes.

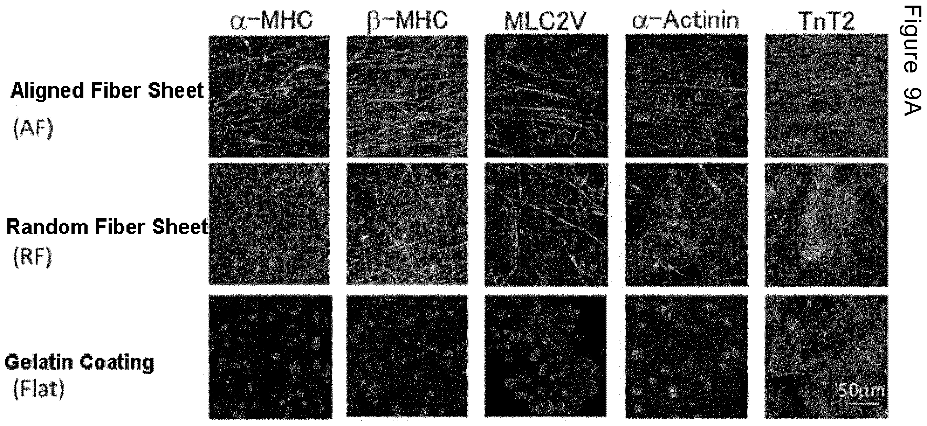

[Figure 9A] Images of the cardiomyocytes that were cultured on the PLGA aligned fiber sheet (AF), random fiber sheet (RF) and gelatin sheet (Flat) were immunostained with cardiomyocyte markers α-MHC, β-MHC, MLC2V, TnT2 and α-Actinin. The images were merged with the images of the fibers in the sheets and DAPI stained signals.

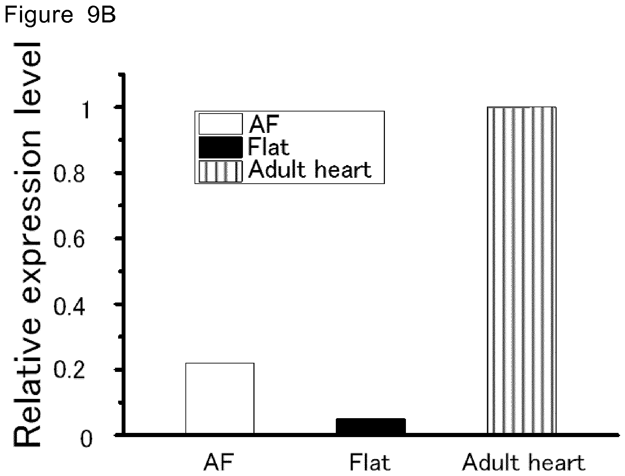

[Figure 9B] Relative expression levels of β-MHC RNA determined by RT-PCR in the cardiomyocytes cultured under the conditions shown in Figure 9A.

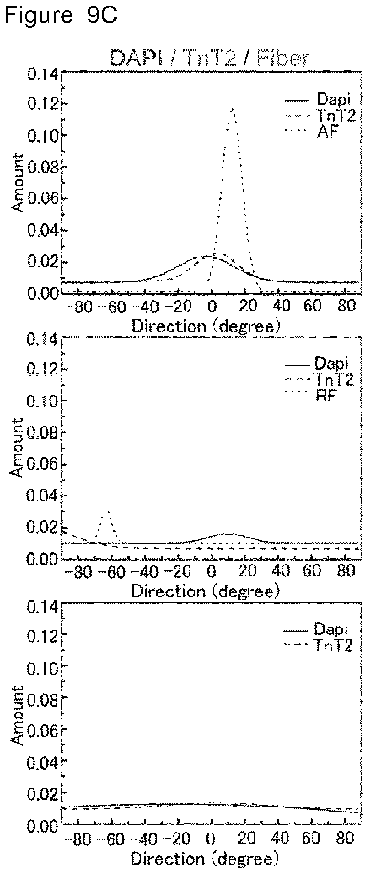

[Figure 9C] Each of TnT2 stain signals shown in Figure 9A were subjected to Fast Fourier transformation. Upper: PLGA aligned fiber sheet (AF), middle: Random fiber sheet (RF), and lower: gelatin coated flat (Flat)

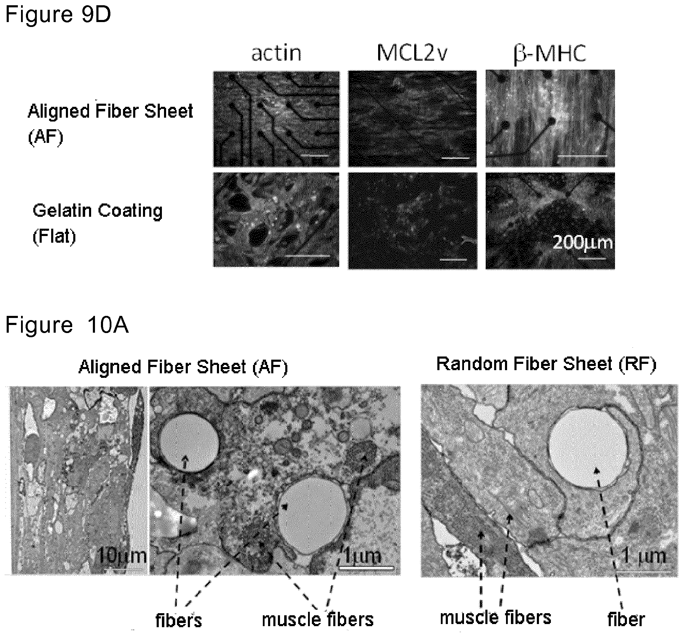

[Figure 9D] Cardiac tissue-like constructs were obtained by culturing cardiomyocytes on the PMGI aligned fiber sheet-coated (AF) and gelatin-coated (Flat) MEAs for 14 days. The tissue-like constructs were immunostained with cardiomyocyte markers, MCL2V and β-MHC as well as actin. The cardiomyocytes cultured on the aligned fiber sheet propagated along the direction of the fibers.
[Figure 10A] Electron microscopic images of the cardiac tissue-like construct prepared on the PLGA aligned fiber sheet (AF) and random fiber sheet (RF).

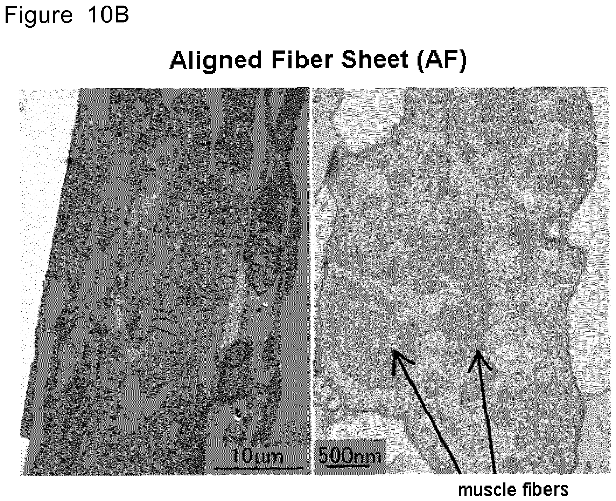

[Figure 10B] Electron microscopic images of quick-freeze, deep-etch preparation of the cardiac tissue-like construct obtained by culturing the cardiomyocytes on the PMGI aligned fiber sheet. Images show the inner part of the cardiac tissue-like construct.

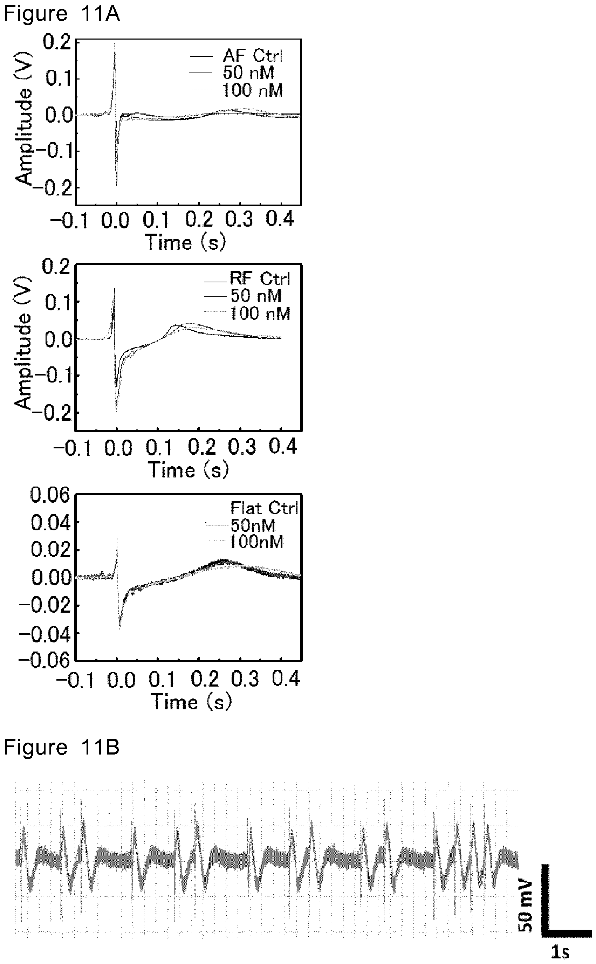

[Figure 11A] QT interval prolongation observed when E-4031 was added to the cardiomyocytes cultured on the PLGA aligned fiber sheet-coated (AF), random fiber sheet-coated (RF) and gelatin-coated (Flat) MEAs.
[Figure 11B] E-4031 was added to the cells cultured on the gelatin-coated MEA and arrhythmia was confirmed 5-10 minutes after the addition of the inhibitor.

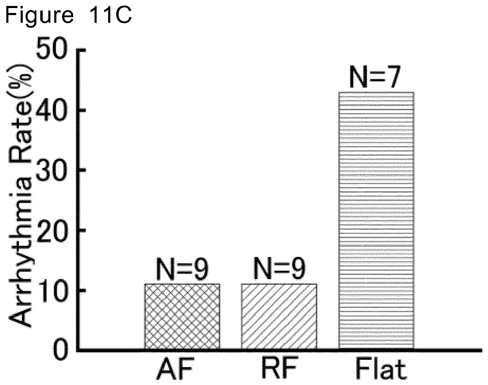

[Figure 11C] Arrhythmia rate (%) observed in each of the cardiomyocyte cultures shown in Figure 11A.

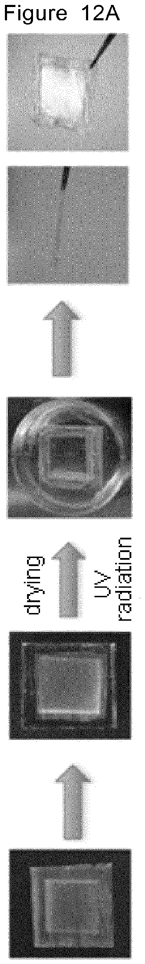

[Figure 12A] A sheet-like cell culture scaffold comprising a PLGA aligned fiber sheet and a frame provided around the aligned fiber sheet, and a cardiomyocyte sheet produced on the cell culture scaffold.

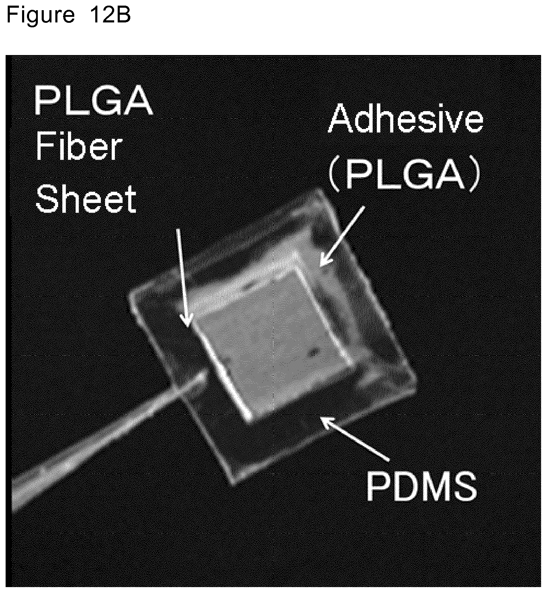

[Figure 12B] A sheet-like cell culture scaffold comprising a PLGA aligned fiber sheet, a PLGA frame that is provided around the fiber sheet and a PDMS spacer adhered to the PLGA frame.

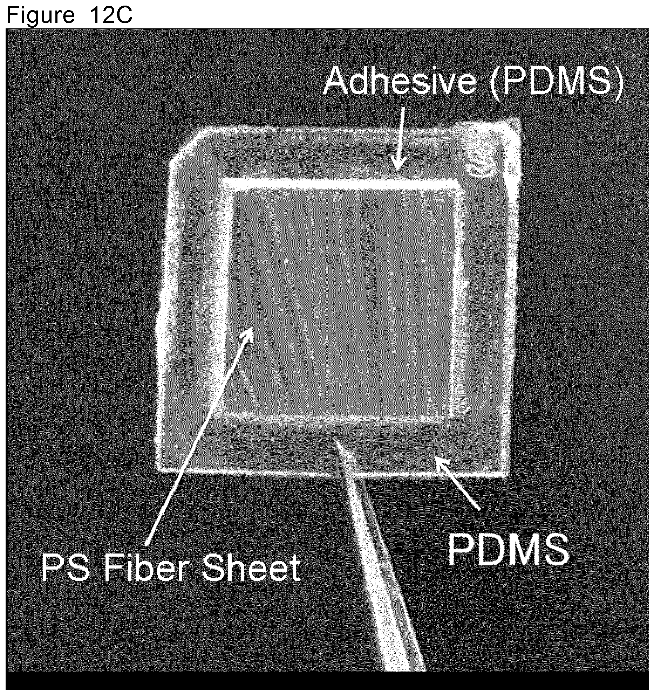

[Figure 12C] A sheet-like cell culture scaffold comprising a PS aligned fiber sheet, a PDMS frame provided around the fiber sheet and a PDMS spacer adhered to the PDMS frame.

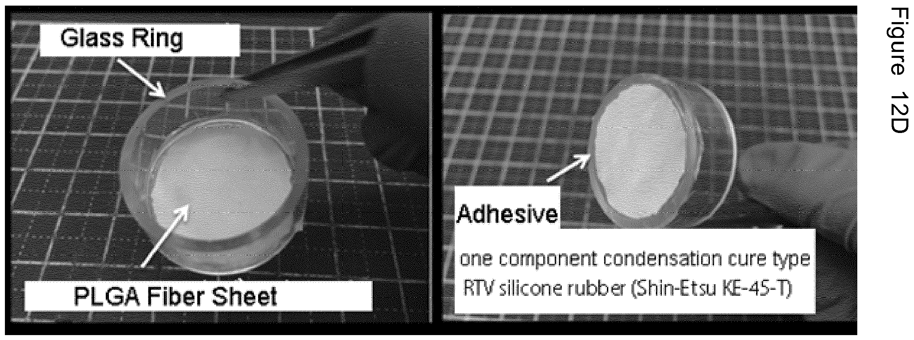

[Figure 12D] A sheet-like cell culture scaffold comprising a PLGA aligned fiber sheet that is adhered to a glass ring with a commercially available adhesive, one-component RTV condensation type silicone rubber.

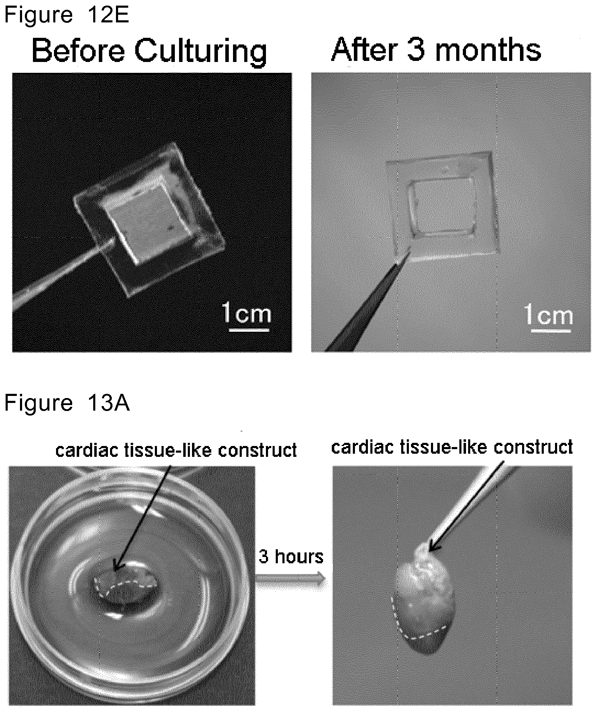

[Figure 12E] Pictures that show the degradation properties of PLGA, a biodegradable material.
[Figure 13A] A mouse heart attached with a cardiac tissue-like construct that was prepared on a PLGA aligned fiber sheet having a PLGA frame.

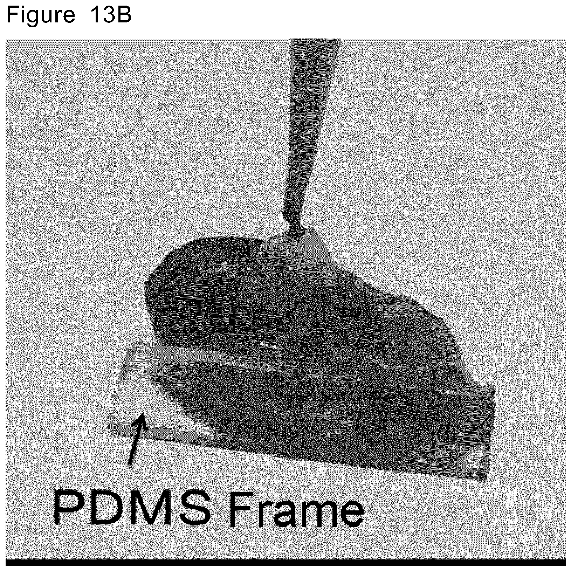

[Figure 13B] A mouse heart attached with a cardiac tissue-like construct that was prepared on a sheet-formed cell culture scaffold having a PLGA aligned fiber sheet and a PDMS frame provided around the fiber sheet. The PDMS frame on the three sides of the aligned fiber sheet was cut off and the tissue-like construct on the PLGA aligned fiber sheet was adhered to the heart.

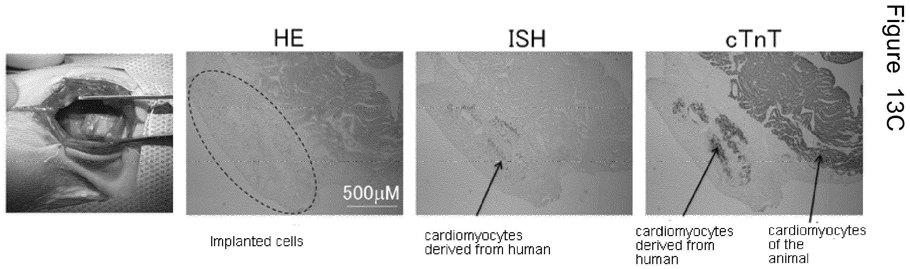

[Figure 13C] A nude-rat heart implanted with cardiac tissue-like construct produced on the PLGA aligned fiber sheet (left). The heart was stained with hematoxyline-eosin (HE), by means of in situ hybridization with a human-specific probe (ISH) and by means of cTnT immunostaining (cTnT).

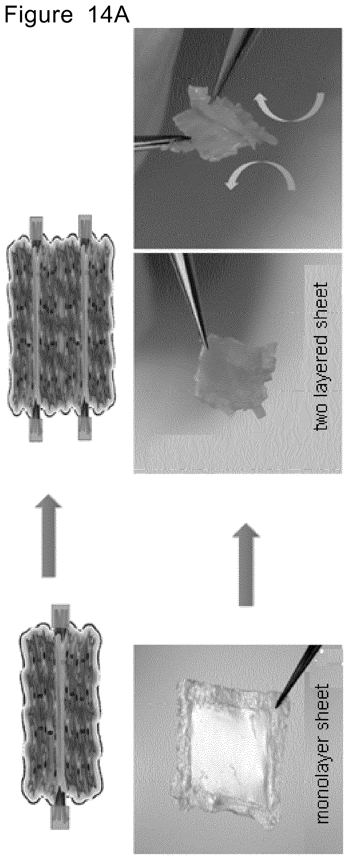

[Figure 14A] Producing a two-layers cardiac tissue-like construct by stacking two cardiomyocyte monolayer tissue-like constructs generated on the PLGA aligned fiber sheet.

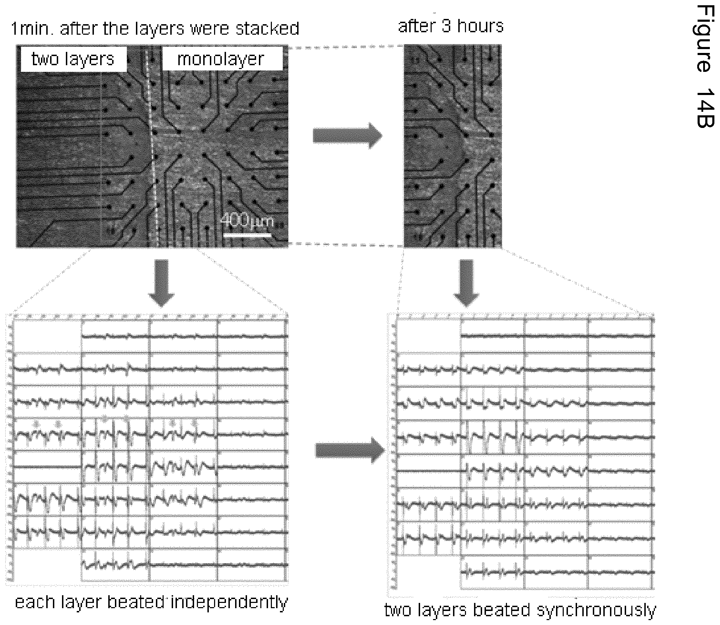

[Figure 14B] Beats from the two layered-cardiac tissue-like construct determined one minute and 3 hours after stacking the layers.

### EMBODIMENTS OF THE INVENTION

According to the present invention, a method for producing an tissue-like construct having an anisotropic structure, for example, cardiac tissue-like construct, which comprises the step of culturing the cells on an aligned fiber sheet. The tissue-like construct manufactured by the method of the present invention may be used for, for example, implantation and evaluation of cellular function by, for example, measuring electric potentials of the cells using a MEA system. Further, the efficacy and safety of a drug candidate substance can be evaluated based on the evaluated cellular function.

The present invention further provides the aligned fiber sheet prepared by the above discussed method, and a tissue-like construct comprising the aligned fiber sheet and cells cultured on the aligned fiber sheet, such as cultured cardiomyocytes. The tissue-like construct of the present invention can be used for implantation. The tissue-like construct can also be used for evaluating cellular function by measuring the electric potentials of the cells with a MEA system and for evaluating efficacy and safety of a drug candidate substance based on the cellular functions.

In the specification and claims, the expression "tissue-like construct" refers to a combination comprising a sheet-shaped cell culture scaffold, such as a fiber sheet and cells cultured on the sheet-shaped scaffold. When the cells constituting the tissue-like construct are cardiomyocytes, the construct may be referred to as "cardiac tissue-like construct". In view of the fact that the tissue-like construct is made from the cultured cells, the tissue-like construct may be referred to as "re-generated tissue-like construct" and "cardiac tissue-like construct" may also be referred to as "re-generated cardiac tissue-like construct".

The fiber sheet used in this invention is a sheet made of integrated fibers. The fibers may be integrated in a random manner or in an aligned manner, especially, aligned so that the fibers are aligned in the same direction.

In this specification, "random fiber sheet" refers to a sheet composed of randomly integrated fibers. "Aligned fiber sheet" refers to a fiber sheet composed of integrated fibers that are aligned in the same direction. Specifically, the aligned fiber sheet represents a sheet wherein equal to or more than 60% of all fibers constructing the sheet are aligned in the direction of ±20° with respect to the fiber orientation (0°). Preferably, equal to or more than 70%, more preferably equal to or more than 80% and especially equal to or more than 90% of all fibers constituting the sheet are aligned in the direction of ±20° with respect to the fiber orientation (0°).

The fibers are made from a polymer. Examples of polymers used for producing the fibers may be any polymer that will not significantly affect the proliferation and physiological activities of cells. The polymer may be either of a biodegradable polymer or a polymer which is hardly degradable in the living body, depending on the use of the fiber sheet. For example, biodegradable polymers may preferably be used for manufacturing a tissue-like construct such as a cellular sheet for use in implantation. On the other hand, highly strong polymers suitable for long term cell culture may be preferably used for producing a tissue-like construct for use in a drug screening and/or cardiotoxicity test.

As biodegradable polymers, materials that have already been used in the medical field are preferably used. A material whose safety has already been confirmed and has already been approved for use as a biodegradable medical material in any of the developed countries is particularly preferable. Examples of biodegradable polymers may include, but are not limited to, polyvinyl alcohol (PVA), polyglycolic acid (PGA), polybutyric acid (PLA), polyethylene glycol(PEG), polyethylene vinyl acetate (PEVA), polyethylene oxide (PEO) and copolymer of polylactic acid and polyglycolic acid (PLGA). PLGA is a material which is finally decomposed in the living body and is discharged as water and carbon dioxide, and therefore, is particularly preferably used. The decomposition speed of PLGA can be controlled by adjusting the ratio between PLA (polylactic acid) and PGA (polyglycolic acid).

A material for producing the fibers which is hardly degradable in the living body is not limited as long as it can form fibers and does not contain a component which would adversely affect the cell culture. Examples may include, but are not limited to, polystyrene (PS), polycarbonate (PC), polymethyl methacrylate (PMMA), polyvinyl chloride, polyethylene terephthalate (PET), polyamide (PA) and polymethyl glutarimide (PMGI). Polymethylglutarimide (PMGI) and polystyrene (PS) are particularly preferable.

The diameter of the fibers constituting the sheet is not particularly limited and may be, for example, 0.1 to 5 µm, preferably 0.5 to 3 µm, more preferably 1 to 2.5 µm. The diameter of the fibers may vary depending on the polymer to be used, the concentration of the solution of the polymer raw and the manufacturing protocol. Optimum manufacturing conditions may be selected based on the polymer and purpose.

When PLGA or PMGI is used as the polymer, fibers having a diameter of 1 to 1.5 µm are exemplified. When PS is used as the polymer, fibers having a diameter of 2 to 2.5 µm are exemplified.

The aligned fiber sheet used for manufacturing the tissue-like construct of the present invention may have a sheet thickness of 0.1 µm or more, for example 1 µm to 20 µm, and preferably 1 µm to 15 µm. For manufacturing a tissue-like construct for use in implantation, an aligned fiber sheet having a thickness of 5 to 20 µm, and more preferably 5 to 15 µm is suitably used. For manufacturing a tissue-like construct for use in drug screening with MEAs, those having a thickness of preferably 1 to 5 µm, and more preferably 2 to 5 µm are preferably used.

The fiber density per 1mm-width of the aligned fiber sheet may vary depending on the diameter of the fibers used and accordingly, the density of the fibers in the aligned fiber sheet may vary depending on the polymer used. The density of aligned fiber sheet of the present invention may be 10 fibers /mm or more, preferably 30-15,000 fibers/mm, and more preferably 50-13, 000 fibers/mm width. The number of fibers per 1mm-widh of the aligned fiber sheet may be determined by cutting the fiber sheet so as to be perpendicular to the fiber direction and counting the number of the fibers observed in the cross-section per unit length in the perpendicular direction.

For example, an aligned fiber sheet made of fibers having diameters of 1-1.5 µm, for example, an aligned fiber sheet made from PLGA or PMGI may preferably have a fiber density of 150-15000 fibers per 1 mm, and more preferably 200-13000 fibers per 1mm-width. When the aligned fiber sheet is used for producing a tissue-like construct for implantation, the density of the sheet may be 5000-15000 fibers per 1 mm, more preferably 8000-13000 fibers per 1mm-width. When the aligned fiber sheet is used for producing a tissue-like construct for use in functional evaluation of the cells with a MEA, the fiber density of the sheet is preferably 150-1,000 fibers per 1 mm, and more preferably 150-500 fibers per 1mm-width.

For example, an aligned fiber sheet made of fibers having diameters of 2-2.5 µm, such as an aligned fiber sheet made from PS may preferably has a fiber density of 30-1000 fibers per 1 mm, more preferably 50-500 fibers per 1mm and further preferably 50-300 fibers per 1mm-width. When the aligned fiber sheet is used for preparing a tissue-like construct for implantation, the density of the sheet may be 150-1000 fibers per 1 mm, more preferably 200-500 fibers per 1mm-width. When the aligned fiber sheet is used for producing a tissue-like construct for use in functional evaluation of the cells with a MEA, the fiber density of the sheet is preferably 30-100 per 1 mm, and more preferably 50-100 per 1mm-width.

The aligned fiber sheet of the present invention may be produced by electrospinning a solution or suspension containing the polymer which is the fiber material. According to a non-limiting example, rotating drum wrapped with a metal sheet such as an aluminum tape may be used as a fiber collector. The fibers ejected from the nozzle are collected onto the rotating drum to give aligned electrospun fiber sheet.

In this embodiment, an aligned fiber sheet made of fibers with a desired fiber diameter may be obtained by selecting, for example, the polymer and its molecular weight, the solvent, concentration and temperature of the polymer solution, the diameter of the nozzle, the feeding rate of the polymer solution, the speed of the drum rotation, the distance between the nozzle and the collector, and the applied voltage.

The concentration of the polymer solution may vary depending on the type and molecular weight of the polymer and may preferably be 0.1-40 wt%. When the polymer concentration is equal to or more than 0.1 wt%, the fiber integrated sheet can easily be obtained with an excellent productivity. In order to avoid the situation where the viscosity of the polymer solution becomes excessively high and the solution is hardly ejected from the nozzle, the polymer concentration is preferably no more than 40 wt%.

The solvent of the polymer solution is not particularly limited as long as it can dissolve the polymer at the above discussed concentration. For example, acetone, triacetin, dimethylformamide, dimethylacetamide, and tetrahydrofuran may be used as a solvent. In order to prevent the polymer from clogging the tip of the nozzle due to evaporation of the solvent, it is preferable to use a highly volatile solvent. Further, one or more solvents may be mixed to improve the solubility of the polymer or the other properties.

The voltage may be appropriately adjusted depending on the type and physical properties of the polymer used for the electrospinning. A voltage of 0.1-50kv, and particularly, 1kv-40kv is exemplified.

Distance between the nozzle and the collector may be set depending on the type and physical properties of the polymer to be used, and the applied voltage. A distance of 10-1000mm, and particularly 30mm-500mm is exemplified.

The shape and size of the aligned fiber sheet of the present invention is not limited and may be appropriately determined in accordance with the shape and size of the tissue-like construct of interest. For example, when the sheet is used for producing a tissue-like construct for implantation, the shape and size of the fiber sheet may be determined in accordance with the target for implantation. Further, when the sheet is used for producing a tissue-like construct for assays with a multi-electrode array (MEA) system, the size and shape of the sheet may be determined based on the MEA used in the assay.

The types of the cells that can be cultured by the method of the present invention are not particularly limited as long as cells aligned in one direction are desired. Cardiomyocytes are exemplified. The cardiomyocytes may be those differentiated from pluripotent stem cells such as ES cells or iPS cells. Various methods of inducing cardiomyocytes from pluripotent stem cells have been known. The methods described in Patent Literature 1 and Non-Patent Literature 5 are exemplified. Commercially available cardiomyocytes induced from ES cells or iPS cells may also be used.

According to the method for manufacturing a tissue like construct of the present invention, the cells are seeded on the aligned fiber sheet and cultured. The aligned fiber sheet may be placed in a conventional cell culture container and the cells may be seeded on the fiber sheet and cultured in a medium. The aligned fiber sheet may be bound to the bottom of the culture container or the sheet may be floated in the culture medium in the container. In order to bind the fiber sheet onto the bottom of the cell culture container, the fiber sheet may be bound with pressure or bound with an adhesive. For example the fiber sheet may be bound to the bottom of the container with an adhesive in a manner that the sheet can be peeled off later. Adhesive is not particularly limited as long as it does not affect the cell culture, and may be a polymer solution, for example, a solution of the polymer from which the fibers constituting the fiber sheet is made, PDMS, and commercially available biocompatible adhesives such as one-component condensation cure type RTV silicone rubber (Shin-Etsu KE-45-T).

Alternatively, a cell culture chamber may be produced and placed on the aligned fiber sheet, and the cells may be cultured in the chamber. In the present specification and claims, "cell culture chamber" represents a structure that is installed to give a wall for keeping the medium in the chamber. The material from which the chamber is made is not particularly limited as long as it does not adversely affect the cell culture. For example, transparent and non-toxic material which is often used for manufacturing micro-fluid devices, such as polydimethylsiloxane (PDMS) and glass are preferably used. A structure looks like a commercially available cell culture plate, that is, a structure composed of integrated chambers each having the aligned fiber sheet is also exemplified. For example, a structure like a commercially available cell culture plate having 6- or 96-wells wherein the bottom of each well is made of the aligned fiber sheet may also be included in this embodiment.

According to the present invention, cell culture medium and conditions may be selected from those known to the art depending on the cells to be cultured and purpose for the culture. For example, the cells may be cultured by stationary culture or shaking culture. By culturing the cells according to the present invention, the cells will align in the direction of the fibers of the aligned fiber sheet to give a tissue-like construct in which the cells are aligned in one direction.

For example, conditions for culturing cardiomyocytes that were induced from iPS cells may be those shown below:
Cell Seeding: 1-100×10⁵ cells/cm²
Culture medium: IMDM medium supplemented with 20% FBS (see Non-Patent Literature 5 and example 2 below) or IMDM medium supplemented with 0.04-0.4% albumin (see Non-Patent Literature 5 and example 2 below).
Culture conditions: Cells are cultured in a 5% CO2 incubator. The medium may be exchanged appropriately.

For obtaining a cardiac tissue-like construct for implantation, the cells may be cultured under the conditions discussed above for 4-10 days.

When cardiomyocytes are seeded and cultured according to the present method, a cardiac tissue-like construct composed of cardiomyocytes aligned in a structure similar to the cardiac tissue in the living body can be prepared. The cardiomyocytes that are contained the cardiac tissue-like construct prepared by the method of the present invention expresses increased level of β-MHC, a cardiac maturity marker. In addition, the electrophysiological activities of the cardiomyocytes in the tissue-like construct were assessed with a MEA system and confirmed QT intervals similar to those obtained from the heart in the living body.

In this specification and claims, the expression of culturing cells "on the fiber sheet" covers both embodiments wherein the cells are cultured only on one side of the sheet and wherein the cells are cultured on both sides of the sheet. The cultured cells may penetrate between the fibers constituting the fiber sheet. Further, in this specification and claims, cultured cells "on the fiber sheet" or tissue-like construct "on the fiber sheet" may represent both embodiments wherein the cells are only on one side of the sheet and wherein the cells are on both sides of the sheet. The cultured cells may penetrate between the fibers constituting the fiber sheet.

In order to culture the cells on both sides of the sheet, a fiber sheet equipped with a spacer around the sheet may be employed. In this specification and claims, "spacer" represents a part provided to the cell culture sheet so that the surface of the sheet does not contact with the bottom of the culture container. The material from which the spacer is made may be any material that does not adversely affect to the cell culture and can be peeled off easily from the fiber sheet. Polydimethylsiloxane (PDMS) and glass are exemplified as the material for spacer. The procedure to attach a spacer to a fiber sheet is not limited and any of known procedures may be employed. For example, the spacer may be attached to the fiber sheet with pressure or with an adhesive that does not adversely affect the cell culture and can be peeled off from the fiber sheet later. Adhesive to be used here is not particularly limited as long as it does not affect the cell culture and can be peeled off from the fiber sheet, and may be a polymer solution, for example a solution of a polymer from which the fibers constituting the fiber sheet is made, PDMS and commercially available biocompatible adhesives such as one-component condensation cure type RTV silicone rubber (Shin-Etsu KE-45-T).

When the aligned fiber sheet equipped with a spacer is used, the cells may be seeded on the one side of the sheet or may be sequentially seeded on the both sides of the sheet. In the latter case, the sheet equipped with a spacer is placed on the bottom of a culture container so that the side with the spacer is up. The cells are seeded on the up side of the sheet so that the cells attach to the sheet. After the seeded cells attach to the sheet, the sheet is reversed and the cells are seeded on the other side of the sheet. A tissue-like construct having cell layers on the both sides of the fiber sheet may be prepared by, for example, culturing the cells with shaking the cell culture container. The method may provide 3D tissue-like construct having cell layers on the both sides of the fiber sheet.

A plurality of tissue-like constructs having cultured cell layers on both sides of the aligned fiber sheet may be stacked. Specifically, two or more tissue-like constructs having cultured cell layers on both sides of the fiber sheet are prepared. The spacers are peeled off from each of the aligned fiber sheets. Then, the two or more tissue-like constructs are stacked so that the directions of the aligned fibers contained in the tissue-like constructs are same. Alternatively, two or more tissue-like constructs obtained by shake culture may be stacked. By culturing the stacked tissue-like constructs, the upper and lower tissue-like constructs are combined and integrated to give a thick 3D tissue-like construct composed of multiple cell layers, for example, a cardiac tissue-like construct.

According to the method of the present invention, a tissue-like construct, for example cardiac tissue-like construct, having 10 or more cell layers can be prepared. Thus obtained tissue-like construct having the 3D structure with anisotropic multiple cell layers has voids between the multilayer structures due to the fiber sheet. Due to the voids between the layers, nutrients reach the cells inside the multilayer structure and therefore, restrictions for possible thickness of the tissue-like construct is very few.

The quality of the tissue-like construct obtained by the method of the present invention, for example, the degree of cell maturity, may be evaluated by an assay such as immune staining. Upon conducting the assay, the tissue-like construct may be adhered to a cell culture container or glass substrate. For example, cardiomyocytes induced from iPS cells are used in the method of the present invention, a cardiac tissue-like construct composed of cardiomyocytes aligned in one direction strongly expressing β-MHC, a cardiomyocyte marker, can be obtained.

The present application also provides the cardiac tissue-like construct generated by the method of the present invention. The cardiac tissue-like construct may be those composed of cardiomyocytes whose β-MHC expression level is 5% or more, 10% or more, or 15% or more, and for example, about 20% of that of adult human normal cardiomyocytes.

The present application further provide a method for manufacturing a tissue-like construct, further comprising the step of evaluating the function of the construct, such as a cardiac tissue-like construct, by detecting the electrical signals from the construct with a MEA contacted to the tissue-like construct.

The present application further provides a method for evaluating the function of the cultured cells, which comprises the steps of contacting the tissue-like construct such as a cardiac tissue-like construct with a MEA and detecting the electrical signals from the tissue-like construct.

The multiple electrode array (MEA) system that can measure the extracellular potential of the cultured cells may be any of the commercially available devices such as a multiple electrodes array system manufactured by multi-channel system MCS GmbH (Germany) and a microelectrode array (MED) system manufactured by Alpha Med Scientific Inc. (Japan). MEA system is a system that can simultaneously observe electrical signals from a plurality of cells by a large number of microelectrodes arranged on a substrate, or can electrically stimulate the cells or tissue and observe the response to the stimulation of the cells. By using a MEA, functions of the cells can be assessed without damaging the cells

With the cardiac tissue-like construct of the present invention, it is expected to evaluate cell functions in a manner close to those observed in the living body. In particular, the waveform of the action potential of the cells is measured with the MEA system and the functions of the cardiomyocytes are evaluated based on the number of the channels that can detect the signals, beating rate, amplitude of the potential, QT intervals, T-wave detection ratio, and incidences of arrhythmia.

In the specification and claims, "multi-electrode array" or "MEA" represents an array of a plurality of microelectrodes, for example 64 microelectrodes are arranged on a substrate.

Effects of an electrical or chemical stimulation given to the cardiomyocytes can be observed by detecting the cardiomyocyte function evaluated by contacting the cardiac tissue-like construct of the present invention with a MEA. In addition, the tissue-like construct of the present invention may also be used for evaluating a drug candidate substance regarding the risk of inviting lethal arrhythmia.

The electrical signals from the tissue-like construct of the present invention produced on the aligned fiber sheet, such as cardiac tissue-like construct may be recorded by contacting the construct with a MEA. In this embodiment, the tissue like construct, for example a cardiac tissue-like construct having an aligned fiber sheet may be contacted with a MEA. Alternatively, cells are cultured and proliferated in a chamber provided on an aligned fiber sheet to give the tissue-like construct and then, the tissue-like construct with the chamber may be contacted with an MEA. The cells strongly attach to the aligned fiber sheet and the sheet is strong, and therefore, the tissue-like construct can easily be handled.

According to the present invention, the tissue-like construct, for example, a cardiac tissue-like construct for implantation may be evaluated with a MEA system. For example, tissue-like constructs for evaluation may be manufactured in parallel with manufacturing a tissue-like construct for implantation under the same conditions. The extracellular potential of the constructs for evaluation may be measured over time and when the tissue-like construct for evaluation reaches a suitable mature level, the construct for implantation may be used for implantation.

Alternatively, the tissue-like construct for implantation may be manufactured and the function of the construct may be evaluated with a MEA before the implantation to confirm the condition of the construct is good. In this embodiment, a plurality of tissue-like constructs may be manufactured at the same time and the best one may be selected and used for the implantation.

For example, when a cardiac tissue-like construct for implantation is manufactured, the timing for implantation may be determined based on the maturity degree of the cells confirmed by, for example, QT interval of the cells as an index. The cardiac tissue-like construct manufactured by the present invention may be confirmed by using a MEA that the construct unlikely occur arrhythmia.

The tissue-like construct may be manufactured on an aligned fiber sheet adhered on a MEA and the cellular function of the tissue-like construct may be evaluated by the electrical signals measured by the MEA. In this embodiment, the action potential of the culture cells is measured upon culturing said cells and therefore, the function of the cells can be evaluated at the same time of culturing the cells.

In this embodiment, the MEA on which the aligned fiber sheet for cell culture is adhered may be placed in a cell culture container and cells are cultured on the aligned fiber sheet. Alternatively, a cell culture chamber may be placed on the aligned fiber sheet adhered on the MEA, and the cells are cultured in the chamber.

The present invention further provide a device for evaluating functions of culture cells, comprising a MEA and a fiber sheet adhered on the array placed on the MEA. The fiber sheet adhered on the MEA may be a random fiber sheet or an aligned fiber sheet.

When the fiber sheet in the device is an aligned fiber sheet, the properties of the aligned fiber sheet, for example, thickness of the sheet, diameter of the fibers and density of the fibers may be similar to those of the above explained aligned fiber sheet.

The device for evaluating cellular functions may be placed in a cell culture container and the cells may be cultured in the container. Alternatively, the device has a cell culture chamber placed on the fiber sheet that is adhered on the MEA and the cells may be cultured in the chamber. The device may be the one like a commercially available cell culture plate in which a plurality of the chamber-having devices are integrated in one plate.

Types of the cells that can be evaluated by the device of the present invention are not specifically limited. When the fiber sheet on the MEA is an aligned fiber sheet, the device may suitably be used for evaluating functions of cells that constitute an aligned structure in the living body, such as cardiomyocytes. The cardiomyocytes maybe those induced from pluripotent stem cells, such as ES cells or iPS cells.

The cell culture medium and cell culture conditions may be appropriately selected from known cell culture procedures based on the cells to be cultured and purpose for manufacturing the tissue like construct. By culturing cells on a MEA on which an aligned fiber sheet is adhered, the cells proliferate along the aligned fibers to give a layer of aligned cells on the MEA.

When cardiomyocytes are seeded on the device of the present invention and cultured, a layer of aligned cardiomyocytes will be formed on the MEA. The cardiomyocyte layer formed on the aligned fiber sheet coated-MEA has a structure similar to those in the living body compared to conventionally prepared randomly propagated cardiomyocyte layer or cardiomyocyte aggregates and is expected to have similar functions as the cardiomyocytes in the living body.

The device according to the present invention may be provided as a device containing cultured cells, such as cultured cardiomyocytes in the cell culture chamber.

The device according to the present invention may preferably be used in various stages of drug developments. For example, the device can be used for evaluating effectiveness and/or safety of drug candidate substances in drug screening.

Accordingly, the present invention also provides a method for evaluating a function of cells, which comprises the step of detecting the electrical signals from the cells, such as cardiomyocytes cultured on the aligned fiber sheet in the device by the MEA. The method of the present invention may further comprise the step of culturing the cells on the aligned fiber sheet in the device.

The cardiomyocytes cultured on the device of the present invention were confirmed highly matured based on the expression of β-MHC, a maturity marker, and the observation of QT intervals. In addition, when the cardiomyocytes are cultured on the device of the present invention, the cultured cells adhere on the MEA firmly. Therefore, stable assay can be conducted with the device of the present invention, i.e. the cells can be cultured for longer time period, provide significantly larger number of channels that can detects the electrical signals, and significantly less likely occur arrhythmia than the cells cultured on a MEA without the aligned fiber sheet of the present invention.

The present application further provides a method for evaluating a function of cells, which comprises the steps of adhering an aligned fiber sheet on a MEA, culturing the cells on the aligned fiber sheet and detecting electrical signals from the cells with the MEA.

The present application further provides a method for evaluating a function of cultured cells, which comprises the step of detecting electrical signals from the aligned cells cultured on an aligned fiber sheet with a MEA contacted with the cells. In this method, the cells may be cultured on the device of the present invention, for example, in a chamber provided on the aligned fiber sheet. Alternatively, the aligned cells that are obtained by culturing the cells on an aligned fiber sheet, for example, in a cell culture chamber provided on the aligned fiber sheet may be contacted with the MEA.

According to the present invention, a sheet-shaped cell culture scaffold having a sheet-shaped cell culture area and a frame provided around the cell culture area. The sheet-shaped cell culture scaffold may be used for manufacturing a tissue-like construct, for example, a construct that can be used for implantation or for drug screening.

In the specification and claims, a "sheet-shaped cell culture scaffold" represents a sheet-shaped substrate having a cell culture area that can be used for manufacturing the tissue-like construct.

In the specification and claims, "sheet-shaped cell culture area" represents a part of the sheet-shaped cell culture scaffold having a sheet-shaped structure on which the cells are cultured.

The material from which the cell culture area is made and the shape of the cell culture area may be selected so that the cells to be cultured can attach and proliferate on the area. Materials that have been known for cell culture scaffold can be used. For example, a sheet of a polymer that has been used as a cell culture scaffold, a non-woven polymer sheet, gel sheet, and a nano-engineered substrate. The polymers used for manufacturing the cell culture area may be any of the polymers that were explained as polymers for preparing the aligned fiber sheet. The material and the surface structure of the sheet-shaped cell culture area may be determined based on the cells to be cultured.

The sheet-shaped cell culture area in the cell culture scaffold may be a fiber sheet obtained by integrating fibers made from a polymer. The fiber sheet may be a random fiber sheet or an aligned fiber sheet.

The material from which the fiber sheet is made may be a biodegradable material or a material that is hardly degradable in the living body, based on the use of the sheet-like cell culture scaffold. For example, when the cell culture scaffold is used for manufacturing a tissue-like construct for implantation, the sheet made from a biodegradable material may be preferably used. When the sheet-like cell culture scaffold is used for drug screening and/or drug safety test, a strong material suitable for long term culture is preferably used.

When the cell culture area of the sheet like cell culture scaffold is made of an aligned fiber sheet, the aligned fiber sheet may have the above-discussed properties, such as thickness, diameter and density.

In the specification and claims, "frame" refers a part provided around the sheet-shaped cell culture area. The frame can be grasped with a pair of tweezers. Accordingly, the sheet-shaped cell culture scaffold can be handled without damaging the fiber sheet area in the scaffold. In addition, the tissue-like construct produced on the sheet-like cell culture scaffold with a frame can be handled without damaging the tissue-like construct.

The width of the frame of the sheet-like cell culture scaffold is not limited and may be determined depending on the use of the sheet-like cell culture scaffold. The width of the frame may be, for example, 2 mm-2 cm, in particular, about 5 mm-1.5 cm.

The material from which the frame is made is not specifically limited and may be a material that does not significantly affect the cell proliferation and cell function, and is strong enough to be handled with tweezers or the like. The material constituting the frame may be a biodegradable material or a material that is hardly degradable in the living body. The material of the frame may be the same or different from the material of the cell culture area. The frame may be provided after preparing the cell culture area, such as a fiber sheet, by applying the polymer around the fiber sheet to give a desired frame width and curing the polymer.

The shape and size of the sheet-like cell culture scaffold of the present invention are not specifically limited and may be determined based on the shape and size of the desired tissue-like construct. A tissue-like construct for use in implantation may be configured based on the part in the living body to be implanted. When the tissue-like construct is for use in an assay with a MEA, the size of the sheet-like cell culture scaffold may be determined according to the MEA to be used.

The sheet-like cell culture scaffold of the present invention may be equipped with a spacer on the sheet, for example, on the frame. The material from which the spacer is made is not specifically limited as long as the material does not significantly affect the cell culture and can be peeled off easily from the frame. Polydimethylsiloxane (PDMS), a transparent and non-toxic material that is known to be used for manufacturing microelectromechanical systems, is exemplified.

The width of the spacer is not specifically limited as long as the spacer supports the sheet. The height of the spacer may be determined based on, for example, the desired thickness of the tissue-like construct and the size of the culture container.

The procedure to adhere the spacer on the frame is not specifically limited and the spacer may be adhered to the frame with pressure, with a polymer solution, for example a polymer solution used for preparing the fibers or bound with an adhesive, for example, an adhesive that can be peeled off later and does not affect the cell culture. Examples of the biocompatible adhesives may include commercially available biocompatible adhesives such as one-component condensation cure type RTV silicone rubber (Shin-Etsu KE-45-T).

The types of the cells that can be cultured by using the sheet-like cell culture scaffold of the present invention are not particularly limited. For example, when a cell culture area is made of an aligned fiber sheet, cells may be those desired to be aligned in one direction. Cardiomyocytes are exemplified. The cardiomyocytes may be those differentiatedfrom pluripotent stem cells such as ES cells or iPS cells. Various methods of inducing cardiomyocytes from pluripotent stem cells have been known. The methods described in Non-Patent Literature 5 and Patent Literature 1 are exemplified. Commercially available cardiomyocytes induced from ES cells or iPS cells may also be used.

According to the method of the present invention, cells are seeded on the cell culture area and cultured. For example, the sheet-like cell culture scaffold of the present invention may be placed in a conventional cell culture container. The cells are seeded on the sheet and cultured in a cell culture medium. The sheet-like cell culture scaffold may be bound on the bottom of the cell culture container in a manner that the sheet can be peeled off from the container after the culture is completed. For example, the sheet may be adhered on the bottom of the container with pressure, or with an adhesive that can be peeled off later. Alternatively, the sheet-like cell culture scaffold of the present invention may be floated in the medium and cells may be cultured on the scaffold.

The cells are seeded on the sheet-like cell culture scaffold and cultured. The cell culture medium and culture conditions may be selected from known cell culturing procedures based on the cells to be cultured and purpose of the culture. The culture may be standing culture or shaking culture. In the tissue-like construct to be prepared on the sheet-like cell culture scaffold of the present invention, cells are not proliferated on the frame area of the sheet-like cell culture scaffold. The frame area in the tissue-like construct can be cramped by tweezers and therefore, the construct can be handled without giving damage to the cells constituting the tissue-like construct.

When the cell culture area is made of an aligned fiber sheet, the cultured cells proliferate along with the direction of the aligned fibers to produce a tissue-like construct having cells aligned in one direction.

When cardiomyocytes are seeded and cultured on a sheet-like cell culture scaffold whose cell culture area is made of an aligned fiber sheet, cardiac tissue-like construct in which the cells are aligned in one direction, i.e. organized in a similar manner in the living body, can be prepared. In the cardiomyocytes constituting the cardiac tissue-like construct obtained by the invention, an elevated expression of a cardiac specific maturity marker β-MHC was confirmed. The action potentials of the tissue-like construct were measured on the MEA and confirmed that the recorded QT intervals were similar to those of a normal heart in the living body.

The present invention further provides a tissue-like construct which comprises the sheet-like cell culture scaffold of the present invention and cells cultured on the scaffold. In particular, the present invention provides a tissue-like construct comprising the sheet-like cell culture scaffold and cells cultured on the cell culture area of the scaffold.

The present invention further provides a method for preparing a tissue-like construct which comprises the step of culturing cells on the sheet-like cell culture scaffold of the present invention.

For example, a cardiac tissue-like construct that is obtained by culturing cardiomyocytes using a sheet-like cell culture scaffold whose cell culture area is made of an aligned fiber sheet contains cells that are organized in an aligned manner. That is, the cardiomyocytes in the tissue-like construct are organized in a manner similar to those in the living heart. The structure of the cardiac tissue-like construct of the present invention is very close to that of the organ in the living body.

The tissue-like construct obtained by using the sheet-like cell culture scaffold of the present invention has a frame area to which the cells do not attach and therefore, can be handled easily without damaging the cells. The tissue-like construct of the present invention can facilitate the operation of tissue implantation.

The tissue-like construct containing a sheet-like cell culture scaffold whose frame area is composed of a biodegradable material can be used for implantation without trimming the frame area, or the frame area may be trimmed before the implantation. The frame area other than the area for pinching with tweezers or the like may be trimmed from the tissue-like construct before implantation. The cell layer in the tissue-like construct is applied to the subject and then, the remaining frame area may be trimmed. By taking those procedures, the efficiency of the operation of tissue implantation will be improved. When the frame is made from a material that is hardly degradable in the living body, the frame area may be trimmed before implantation.

The tissue-like construct prepared by using the sheet-like cell culture scaffold of the present invention may include a construct having cultured cells on both sides of the sheet-like cell culture area. The tissue-like construct having cultured cells on both sides of the cell culture area may be prepared by using a sheet-shaped cell culture scaffold equipped with a spacer. When the sheet-shaped cell culture scaffold equipped with a spacer is used, the cells may be seeded on the one side of the sheet or may be sequentially seeded on both sides of the sheet one by one. In the latter case, the sheet equipped with a spacer is placed on the bottom of a culture container so that the side with the spacer is up. The cells are seeded on the up side of the sheet so that the cells attach to the cell culture area. After the seeded cells are attached to the cell culture area, the sheet is reversed and the cells are seeded on the other side of the cell culture area. A tissue-like construct having cultured cells on both sides of the fiber sheet may be prepared by culturing the cells with shaking the cell culture container. The method may provide a 3D tissue like construct having cell layers on both sides of the sheet-shaped cell culture scaffold.

A plurality of tissue-like constructs having cultured cell layers on both sides of the sheet-shaped cell culture scaffold may be stacked. In detail, two or more tissue-like constructs having cultured cell layers on both sides of the sheet-shaped cell culture scaffolds equipped with a spacer are prepared. The spacers are peeled off from each of the sheet-shaped cell culture scaffolds. Then, the two or more tissue-like constructs are stacked so that the directions of the alignment of the cells in the tissue-like constructs are same. Alternatively, two or more tissue-like constructs obtained by shake culture may be stacked. By culturing the stacked tissue-like constructs, the upper and lower tissue-like constructs joined together to give a thick 3D tissue-like construct composed of multiple cell layers.

Thus obtained tissue-like construct having the 3D structure with multiple cell layers, for example with 10 or more cell layers has voids between the multilayer structures due to the fibers in the sheet-shaped cell culture scaffold. Due to the voids between the layers, nutrients reach the cells inside the multilayer structure and therefore, restrictions for possible thickness of the tissue-like construct is very few.

For example, cardiomyocytes induced from iPS cells are used for preparing a tissue-like construct using the sheet-shaped cell culture scaffold of the present invention, cardiac tissue-like construct in which the cells strongly express a cardiomyocyte maturity marker β-MHC and are organized in an aligned manner can be obtained. The action potentials of the tissue-like construct measured by a MEA represent QT intervals that are similar to a normal heart in the living body.

A tissue-like construct prepared with the sheet-shaped cell culture scaffold of the present invention may be placed on a MEA to measure the extracellular action potentials of the cells constituting the construct. Accordingly, the present application further provides a method for evaluating function of the tissue-like construct comprising the step of measuring the electrical signals from the construct.

For example, tissue-like constructs for evaluation may be produced in parallel with manufacturing a tissue-like construct for implantation under the same conditions. The extracellular potential of the constructs for evaluation may be measured over time and when the tissue-like construct for evaluation reaches a suitable maturity, the construct for implantation may be used for implantation. In this embodiment, the tissue like construct is placed on the MEA. When cardiac tissue-like construct is prepared, for example, QT interval of the cells may be used as an index for determining the timing for implantation.

Alternatively, the tissue-like construct for implantation may be manufactured and the function of the construct may be evaluated with a MEA before the implantation to confirm the condition of the construct is good. In this embodiment, a plurality of tissue-like constructs may be produced at the same time and the best one may be selected and used for the implantation.

For example, when a cardiac tissue-like construct for implantation is manufactured, the timing for implantation may be determined based on the maturity of the cells confirmed by, for example, QT interval of the cells as an index. The cardiac tissue-like construct produced by the present invention may be confirmed by using a MEA that the construct unlikely occurs arrhythmia.

The present application further provides a product comprising a tissue-like construct prepared with the sheet-shaped cell culture scaffold of the present invention and enclosed in a sterilized package. The product may further comprise cell culture medium in the sterilized package together with the tissue-like construct of the present invention. The packaged tissue-like product is easy to transport.

The packaging material is not specifically limited and is preferably a package that is gas permeable and can provide a closed system, for example, cell culture bag (Takara Bio Inc.).

The tissue-like construct is preferably used for drug screening by using a MEA system. Similar to the above-discussed tissue-like constructs, constructs that exert preferable QT intervals or that do not exert arrhythmia may be selected and used for drug screening.

Accordingly, the present invention provides a method evaluating the function of the cells constituting a tissue-like construct, comprising the step of measuring electrical signal from the tissue-like construct that is produced on a sheet-shaped cell culture scaffold having a sheet-shaped cell culture area and a frame around the area by means of a MEA system. The present invention further provides a method for evaluating the efficacy and/or safety of a drug candidate substance based on the cellular function as an index.

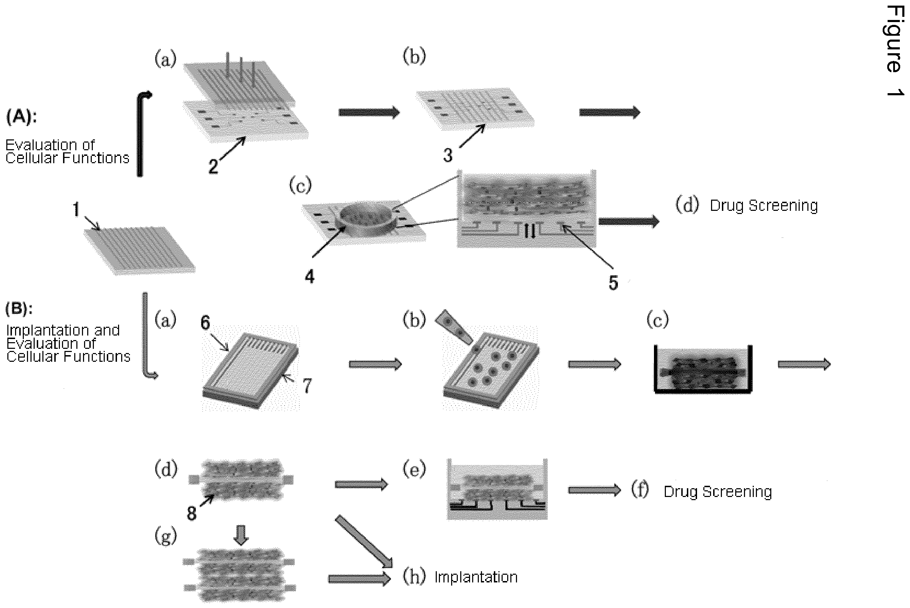

In figure 1, the general idea of this application is shown. The cardiac tissue-like construct produced with an aligned fiber sheet can be used for (A) evaluating cellular functions or (B) implantation and evaluating cellular functions. (A) When the tissue-like construct is used for evaluating cellular functions, the aligned fiber sheet is applied on the electrodes of a multi-electrode array system (MEA) (a). A cell culture chamber is provided on the aligned fiber sheet (b) and cardiomyocytes are cultured in the chamber while electrical signals from the cells are measured (c, real time measurement) . This embodiment can be used for drug screening (d). (B) When the tissue-like construct that can be used for implantation and for evaluating cellular functions, a frame may optionally be provided around the aligned fiber sheet (a), cardiomyocytes are seeded on the fiber sheet (b) . A fiber sheet equipped with a spacer may optionally be used. Cardiomyocytes are cultured in a manner aligned in one direction (c). The functions of the produced cardiac tissue-like construct (d) may be evaluated by contacting the construct with the multi-electrode array (e) and accordingly, may be used for drug screening (f) . A plurality of thus produced tissue-tissue like constructs may optionally be stacked each other to give a multilayer tissue-like construct (g) . Thus prepared tissue like constructs can be used for implantation (h).

Examples for carrying out the present invention are shown below. The present invention is not limited to the specific procedures explained in those examples.

### EXAMPLE 1: Preparation of an aligned fiber sheet by electrospinning

### 1-1. Preparation of a PLGA aligned fiber sheet

### A. Preparation protocol

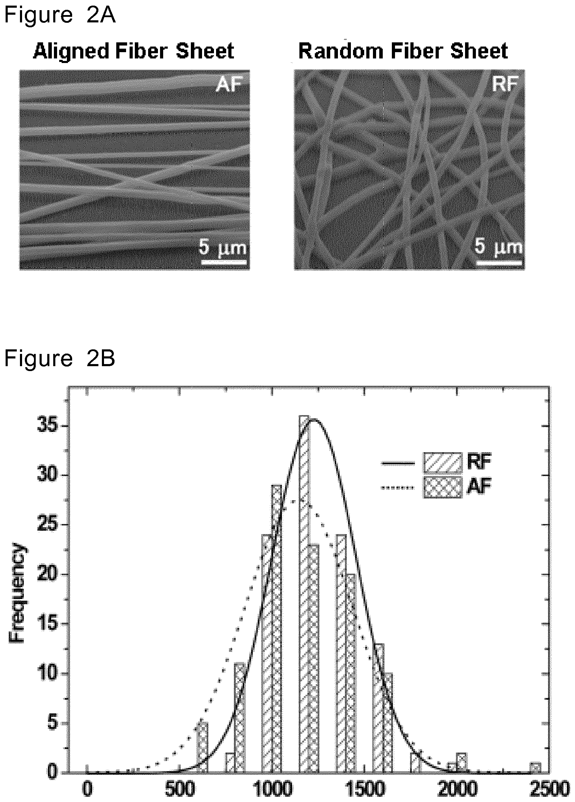

A 20-25% solution of PLGA (P1941 SIGMA, PLA75%:PGA25%, mol wt. 66, 000-107, 000) in THF was prepared. The solution was loaded into a syringe equipped with a 25G blunt needle (Nipro) and air bubbles in the syringe were pulled out. The syringe was set in a micro-syringe pump at a flow rate of 10mL/minute. A layer of aluminum foil tape (Sansyo Co., Ltd.) was attached to the surface of a high-speed rotating drum and the drum was placed so that the distance between the tip of the needle and the drum was 10 cm. The positive electrode of the high-voltage power supply was connected to the needle and the negative electrode was connected to the drum. The high-speed rotating drum was rotated at 3000 rpm. The switch of the microsyringe pump was turned on and the fibers were electrospun under 8kV of voltage. The electrospun fibers were collected on the aluminum foil tape on the drum in an aligned manner. The electrospining was conducted for 40 seconds. After the termination of the electrospinning, the aluminum foil tape on which fibers are collected was removed from the drum to give the aligned fiber sheet. Random fiber sheet (RF) was prepared in a similar manner as above by randomly ejecting fibers for 20 seconds (Figure 2A).

### B. Property of the PLGA fiber sheets

### Diameter of PLGA fibers

High resolution images of the aligned and random PLGA fiber sheets prepared in Example 1-1-A as above were obtained by using an electron microscope (JEM1400; JEOL Ltd. , Japan) and fiber diameters were measured using Image-J software. The distribution of diameters of the fibers is shown in Figure 2B. As shown in Figure 2B, the average diameters of PLGA aligned fiber sheet and PLGA random fiber sheet were 1-1.5 µm.

### Elasticity of the PLGA aligned fiber sheet

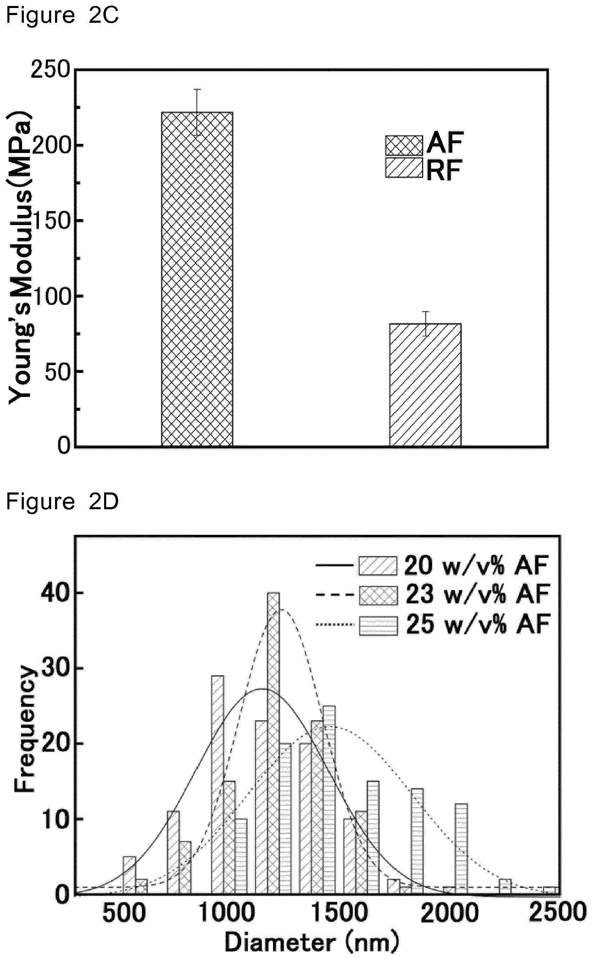

A PLGA aligned fiber sheet (spin time: 40 seconds, thickness: 2 um, fiber Density: 300 fibers/mm) and a PLGA random fiber sheet (spin time: 20 seconds, thickness: 2 µm) were prepared according to Example 1-1-A above. The obtained sheets were subjected to the tensile test using a tensile testing machine (Shimadzu Autograph AGS-X) to evaluate the elasticity of the sheets. Results are shown in Figure 2C. The elasticity (220 MPa) of the aligned fiber sheet in the direction parallel to the aligned fibers was about three times higher than that of the random fiber sheet.

### Concentration of the polymer solution for manufacturing the PLGA fiber sheet

The diameter of the electrospun fibers may be controlled by adjusting the concentration of the polymer in the polymer solution used for the preparation, provided that the other conditions are not altered. In order to determine the optimal polymer concentration under the conditions in above Example 1-1-A, aligned fiber sheets were prepared using PLGA solutions with different PLGA concentrations (20%, 23% and 25%) according to the procedure of Example 1-1-A. The distribution of fiber diameters of the obtained aligned fiber sheets were measured and confirmed that the diameter of the fibers increase as the concentration of the polymer solution increases (Figure 2D). It was confirmed that 20-23% PLGA solution is suitable for stable production of electrospun fibers with a diameter of 1-1.5 µm.

### Thickness and fiber density of the fiber sheet.

Electro microscopic image of the aligned PLGA fiber sheet prepared in Example 1-1-A (spin time: 40 seconds) was obtained by using an electron microscope (JEM1400; JEOL Ltd. , Japan) and the thickness of the fiber sheet was measured. In addition, the number of the fibers per unit length in a cross section cut at right angles to the direction of the aligned fibers was counted to confirm the number of fibers (fiber density) per 1mm of sheet width. The thickness of the PLGA aligned fiber sheet was 2µm and the fiber density was 300 fibers/mm.

### Angular distribution of the fibers in the PLGA aligned fiber sheet

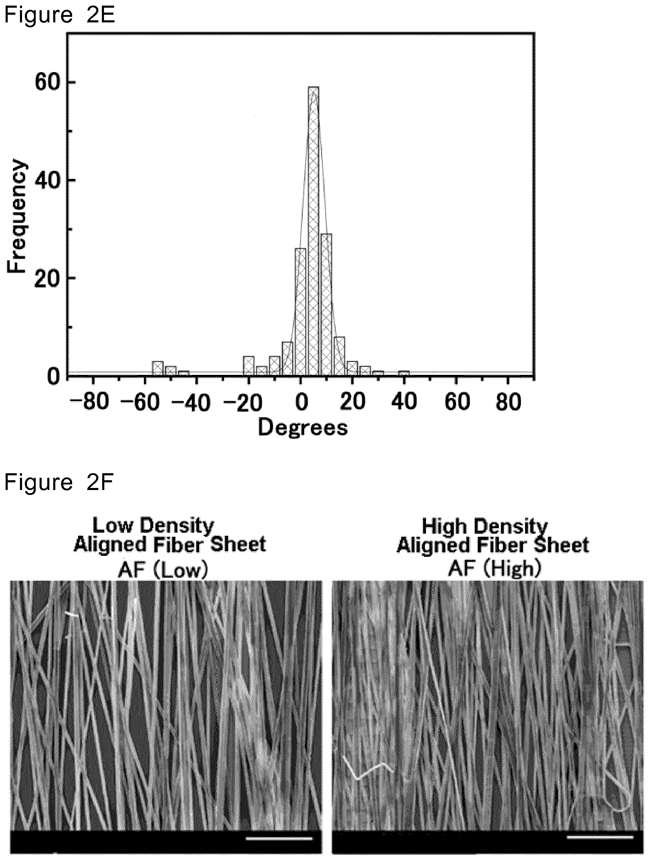

Electromicroscopic image of the PLGA aligned fiber sheet (spin time: 40 seconds) was obtained and analyzed the image with an imaging software (Image J) to measure the angular distribution of the fibers in the fiber sheet. More than 90.8% of the fibers constituting the PLGA aligned fiber sheet were in the direction + 20° or less to the direction of the aligned fibers (0°) (Figure 2E). The thickness of the PLGA random fiber sheet produced according to the procedures of Example 1-1-A was 2 µm.

### 1-2. Preparation of a PMGI aligned fiber sheet.

### A. Preparation protocol

A PMGI aligned fiber sheet was prepared according to the similar procedure as in Example 1-1-A using a 16% PMGI solution (Michro chem) instead of the PLGA solution. According to the electrospinning method, the fiber density in an aligned fiber sheet may be increased by increasing the spin time. Low density PMGI aligned fiber sheet (AF (Low)) and high density PMGI aligned fiber sheet (AF (High)) were prepared by setting the spin times to 90 seconds and 300 seconds, respectively (Figure 2F, the scale bar represents 50 µm).

### B. Properties of the PMGI aligned fiber sheets.

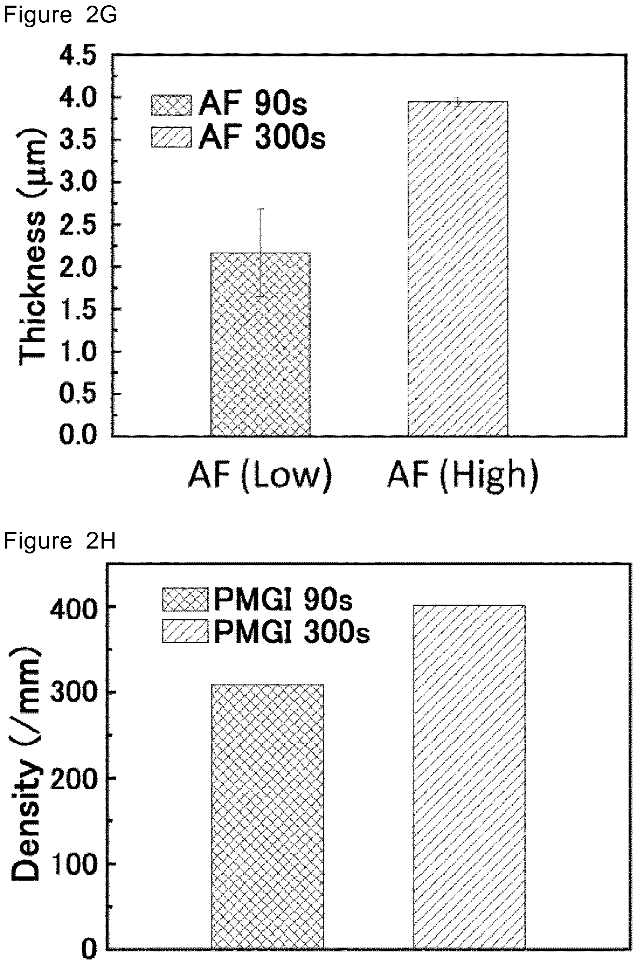

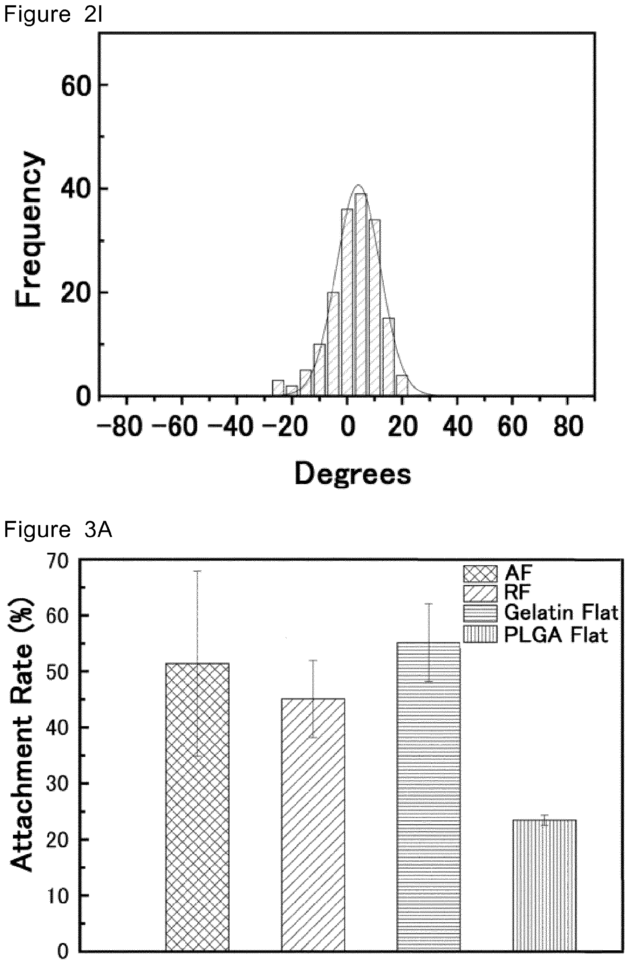

Thickness and fiber density of each PMGI aligned fiber sheets were measured in the same manner as in above Example 1-1. The thickness of the low density PMGI aligned fiber sheet (spin time: 90 seconds) was 2 µm and that of the high density MPGI aligned fiber sheet (spin time: 300 seconds) was 4 µm (Figure 2G). The fiber density of the low density PMGI aligned fiber sheet was 300 fibers/mm and the high density PMGI aligned fiber sheet was 400 fibers/mm (Figure 2H). Angular distribution of the fibers constituting the PMGI low density aligned fiber sheet was measured according to the procedure explained in above Example 1-1 and confirmed that more than 97% of the fibers constituting the aligned fiber sheet were in the direction ± 20° or less to the direction of the aligned fibers (0°) (Figure 2I).

### 1-3. Preparation of polystyrene aligned fiber sheet.

### A. Preparation protocol

A 30% solution of polystyrene (PS: Sigma 182435, mol wt. : 130,000-290,000) in DMF was prepared. The solution was loaded into a syringe equipped with a 27G blunt needle (Nipro) and air bubbles in the syringe were pulled out. The syringe was set in a micro-syringe pump at a flow rate of 1 mL/minute. A layer of aluminum foil tape (Sansyo Co., Ltd.) was attached to the surface of a high-speed rotating drum and the drum was placed so that the distance between the tip of the needle and the drum was 15 cm. The positive electrode of the high-voltage power supply was connected to the needle and the negative electrode was connected to the drum. The high-speed rotating drum was rotated at 1000 rpm. The switch of the microsyringe pump was turned on and the fibers were electrospun under 20kV of voltage. The electrospun fibers were collected on the aluminum foil tape on the drum in an aligned manner. The spin times were 10 minutes and 50 minutes. After the termination of the electrospinning, the aluminum foil tape on which fibers were collected was removed from the drum to give the PS aligned fiber sheets. Nanofiber manufacturing machine, MECC, NF500, Japan

### B. Properties of the polystyrene aligned fiber sheets.

Thickness and fiber density of the above obtained polystyrene aligned fiber sheets were measured in the same manner as in above Example 1-1. The properties of the fibers were confirmed as follows. Low density PS nanofiber sheet: spin time: 10 minutes, thickness: 2 µm, and fiber density: 50 fibers/mm. High density PS nanofiber sheet: spin time: 50 minutes, thickness: 10 µm and fiber density: 300 fibers/mm. In addition, it was confirmed by the similar procedures as those in Example 1-1 that the average diameter of the PS fibers constituting the PS aligned fiber sheets was 2 µm and the distribution of the directions of the fibers was within 0 ± 5°.

### EXAMPLE 2. Cardiomyocyte culture on the aligned fiber sheet. 2-1. Cardiomyocyte culture on PGLA aligned fiber sheet.

### A. Culture of cardiomyocytes induced from human iPS cells (IMR90-1)

Cardiomyocytes induced from human iPS cells (IMR90-1) according to the protocol taught in Patent Literature 1 and Non-Patent Literature 5 were used. The colonies of cardiomyocytes were placed in a 50 ml centrifuge tube and centrifuged at 50G for 2 minutes. The supernatant was removed and the cells were washed once with PBS (Gibco). A protease solution (0.1% collagenase type I (Life technologies), 0.25% trypsin, 1 U/mL DNase I (Applied Biosystems), 116mM NaCl, 20mM HEPES, 12.5mM NaH₂PO₄, 5. 6mM glucose, 5.4mM KCl, and 0.8mM MgSO4 [pH 7.35]) 100 mL was added to the tube together with two stirrer bars that were sterilized by using a gas burner, and the cells were incubated at 37°C with stirring. By this procedure, the cells were dissociated to some extent and the cells were filtered using a 40 µm filter (MilliQ) mounted on another 50 mL tube. 20ml of 20% serum-supplemented IMDM medium (Sigma-Aldrich) (20% FBS (Gibco), 1% MEM nonessential amino acid solution (Sigma), 1% penicillin/streptomycin (Gibco), 2 mM L-glutamine (Sigma), 0.5mM L-carnitine (Sigma), 0.001% 2-mercaptoethanol, 0.005N NaOH and 10ng/mL BMP, hereinafter represented as "20% FBS medium") was gently poured on the filter. Then, the filter was removed from the tube and placed upside down in a 6 cm shale to collect the colonies remained on the filter. The filter was washed with 5mL of protease solution by applying the solution from the back side and the colonies in the shale were returned to the first tube containing the protease solution and stirrer bars, and incubated for additional 20-30 minutes with stirring at 37°C. The cells in the protease solution in the tube were then, filtered through the 40µm filter and the filter was washed with 10 ml of the 20% FBS medium. In the tube equipped with the filter contains 45 ml of cell suspension in total including 15 mL of the protease solution and 30mL of the 20% FBS medium. The number of the cells in the 45mL of the suspension was counted. The cells were centrifuged at 1000rpm for 3 minutes and the supernatant was removed. The 20% FBS medium added to the tube so that the cell density was adjust to a desired value. Namely, the cell was suspended in the 20% FBS medium to give a suspension with a cell density of 5×10⁵ cells per 100 µL medium and then, the cell suspension was further adjusted to a density of 2×10⁵ cells per 100µL. The cells were dispersed in the medium by pipetting.

Glass substrates coated with a PLGA aligned fiber sheet (AF, spin time: 40 seconds, thickness: 2µm) and a PLGA random fiber sheet (RF, spin time: 20 seconds, thickness: 2µm) were used. In addition, glass substrates coated with gelatin (Gelatin Flat) and non-fiber PLGA film (PLGA Flat) were also used. Attachment of cardiomyocytes on each of the substrates was evaluated. 100µL of the cell suspension prepared in above Example 2-1-A was seeded on each of the PLGA aligned fiber sheet-coated (AF), random fiber sheet-coated (RF), Gelatin Flat-coated and PLGA Flat-coated glasses at a density of 2×10⁵ per 1cm² of the substrate. The PLGA fiber sheet is highly hydrophobic and the cell suspension formed oval droplets on the sheet. The substrates were left to stand in a 5% CO₂ incubator at 37°C for 5-15 hours to allow the cells attach to the substrate. The attachment rates of the cells on the substrates were confirmed and the 20% FBS medium were added so that the substrates were fully soaked in the medium. On day 3, the medium was replaced with FBS(-) IMDM medium (IMDM containing 1% MEM nonessential amino acid solution, 1% penicillin/streptomycin (Gibco), 2mM L-glutamine (Sigma), 0.5mM L-carnitine (Sigma), 0.001% 2-mercaptoethanol (Gibco), 1-2% BSA (Wako, Osaka) or 0.4% human serum albumin (Sigma-Aldrich), 4 µM CHIR (Axon) and 2µM BIO(Calbiochem), hereinafter referred to as "FBS(-) medium".

### B. Attachment of the cardiomyocytes

The cells were incubated for 5 hours from the seeding then the cells that were not attached to the substrates were removed and the number of the cells attached to the substrate was counted (Figure 3A). As a result, the cell attachment rates on the PLGA fiber sheet-coated substrates were significantly higher than that of the PLGA film coated substrate (PLGA flat).

### C. Study on the optimal spinning time for manufacturing the aligned fiber sheets.

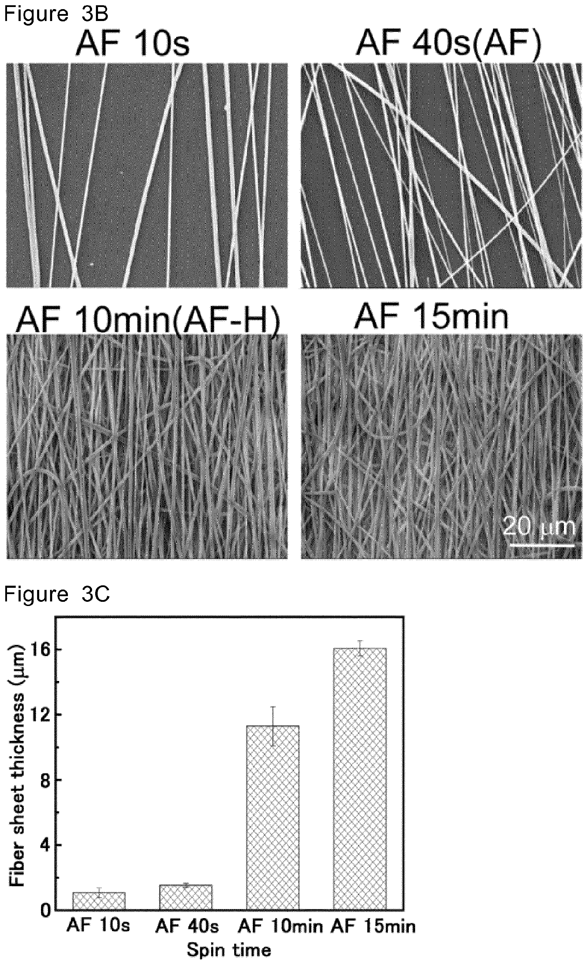

The effects of the fiber density of the aligned fiber sheet on the cell attachment rate were studied. As above described, a fiber sheet with a higher fiber density can be obtained by increasing the time for electrospinning. PLGA aligned fiber sheets were prepared according to Example 1-1-A with spin times of 10 seconds, 40 seconds, 10 minutes and 15 minutes, and electro microscopic images of the obtained sheets were obtained (Figure 3B). Further, the thicknesses of thus prepared fiber sheets were measured according to the procedures of Example 1-1-C. As the fiber density increased, the thickness of the fiber sheet increased from 1 µm to 16 µm.

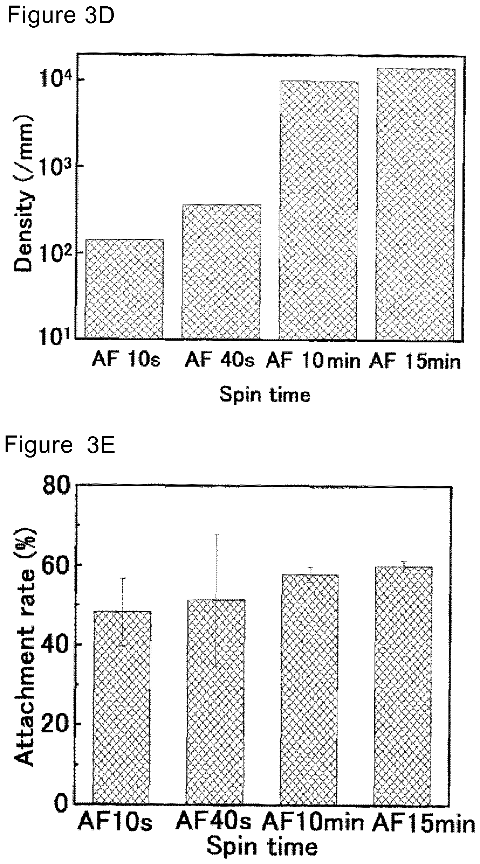

Based on the electromicroscopic images (Figure 3B) and thicknesses (Figure 3C) of the PLGA aligned fiber sheets produced by the different conditions, the fiber densities of the fiber sheets were confirmed and were approximately as follows (Figure 3D):

| | |
|---|---|
| spin time | 10 seconds: 150 fibers/mm |
| | 40 seconds: 300 fibers/mm |
| | 10 minutes: 100,000 fibers/mm |
| | 15 minutes: 150,000 fibers/mm. |

Cardiomyocytes induced from human iPS cells (IMR90-1) were cultured on each PLGA aligned fiber sheet and determined the cell attachment rate according to the procedure of Example 2-2-A. As the fiber density increased, the cell attachment rate increased with good reproducibility. Especially good cell attachment rate were observed when the cells were cultured on the PLGA aligned fiber sheets produced by electrospining of 10 minutes and 15 minutes. On the other hands, when a low fiber density fiber sheet was used, the variation in cell attachment rate was large between samples (Figure 3E).

### 2-2. Cardiomyocyte culture on PMGI aligned fiber sheet.

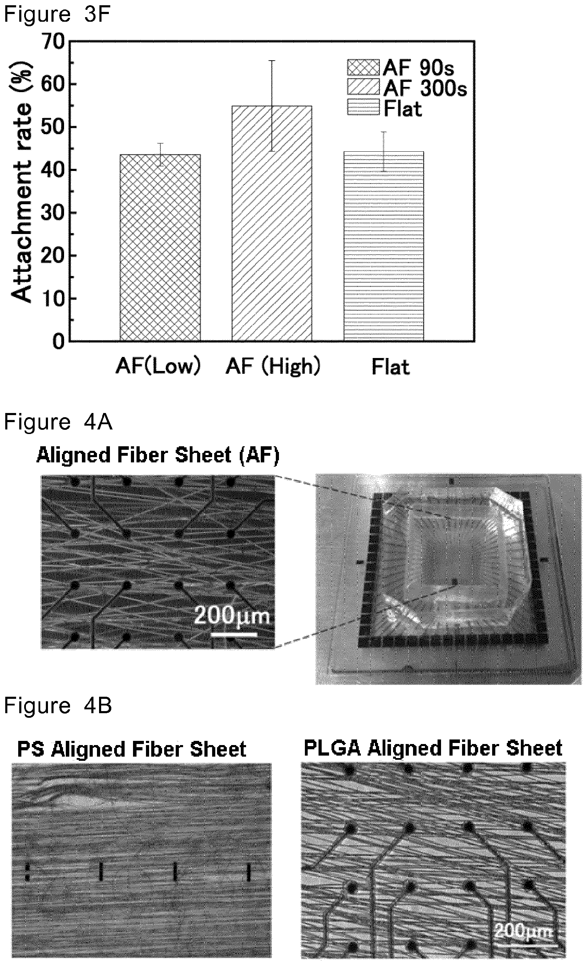

The effect of the fiber density of the aligned fiber sheet on the cell attachment rate was studied with PMGI aligned fiber sheet. Grass substrates coated with low density PMGI aligned fiber sheet (spin time: 90 seconds, thickness: 2µm, fiber density: 300 fibers/mm) and high density PMGI aligned fiber sheet (spin time: 300 seconds, thickness: 4 µm, fiber density: 400 fibers/mm) prepared according to Example 1-2-A, and glass substrate coated with gelatin (FLAT) were used. The ×cardiomyocytes were seeded on each of the substrate, incubated 5 hours, and the cell attachment rate on each substrate was determined according to the procedures of Example 2-1. The cell attachment rates on the PMGI aligned fiber sheet-coated substrates increased as the fiber density increased (Figure 3F). The cell attachment rates of the low density PMGI aligned fiber sheet-coated substrate and gelatin-coated substrate were comparable.

### EXAMPLE 3. Preparation of a device for evaluating cellular function with a multi-electrode array (MEA) system

Electrospun PMGI aligned fibers were integrated on the aluminum foil tape to give an aligned fiber sheet (AF, spin time: 90 seconds, thickness: 2 µm, fiber density: 300 fibers/mm) according to Example 1. The fiber sheet was adhered on a 64 channel MEA (nitride-coated gold electrodes, Multi-Channel Systems, Germany) (electrode size: 30 µm diameter, 200 µm spacing 8 × 8 grid array) with a vise heart press machine (MNP-001, As One Corporation) to give a device for evaluating cellular functions (Figure 4A left). PDMS chamber for cell culture (2cm × 2cm) was prepared and adhered on the PMGI aligned fiber sheet adhered on the MEA (Figure 4A right). The device was dried overnight to evaporate the solvent and sterilized by UV radiation for 30 minutes, then, used for evaluation of cardiomyocyte cellular functions.

Similarly, devices for cellular function evaluation were prepared by adhering a PS aligned fiber sheet (spin time: 10 minutes, thickness: 2 µm, fiber density: 50 fibers/mm) and a PLGA aligned fiber sheet (spin time: 40 seconds, thickness: 2 µm, fiber density: 300 fibers/mm) instead of the PMGI aligned fiber sheet on the MEAs (Figure 4B).

### EXAMPLE 4. Measurement of cardiomyocyteular action potentials with the MEA system

### 4-1. Action Potentials of cardiomyocytes cultured on the PMGI aligned fiber sheet.

### A. Measurement of action potentials of cultured cardiomyocytes.

Cardiomyocytes induced from iPS cells (IMR90-1) were seeded in the PDMS chamber of the device for evaluating cellular functions prepared in Example 3. The cells were seeded and incubated under the conditions according to Example 2. After 5 hours incubation, it was confirmed that the cells were attached firmly on the sheet and then, the chamber was added with the 20% FBS medium. The cells were incubated in an incubator at 37 °C incubator. On day 2 of incubation, the medium was exchanged with the FBS (-) medium and after that, the medium was exchanged with the fresh FBS (-) medium every 4 days. Before the exchange of the medium, the electrical signals were measured. Before the measurement, the device was placed on a heat plate at 37°C and kept there for 5 minutes so that the temperature became stable. After the measurement of the electrical signals, the culture medium was exchanged and the cells were further cultured in the incubator. A softwer MC-RACK provided by Multi-channel systems was used for the measurement of action potentials and OriginPro 9.0 was used for data analysis.

### B. Cardiomyocyte culture on the aligned fiber sheet.

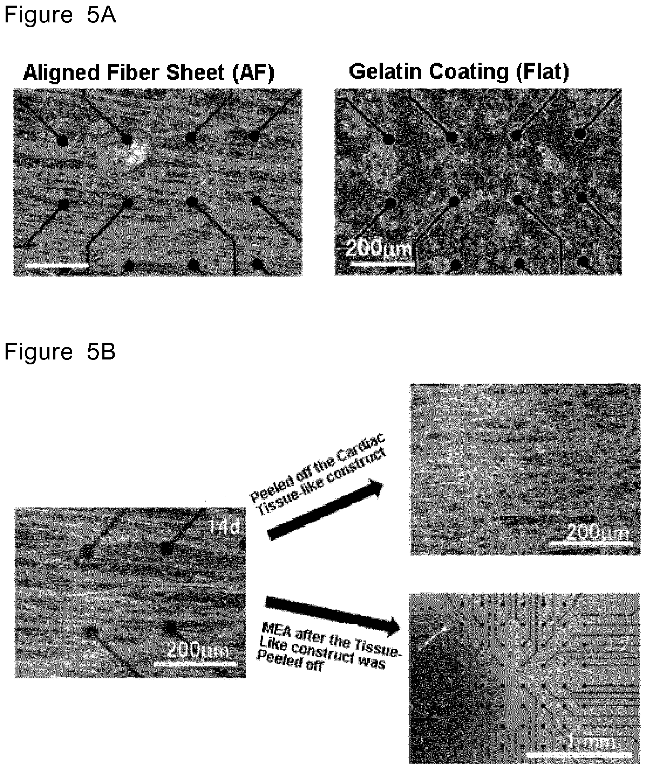

According to the procedure of Example 4-1-A, cardiomyocytes were seeded on the PMGI aligned fiber sheet-coated (AF) and gelatin-coated (Flat) MEAs and cultured. On day 2 of the culture, the cells were microscopically observed and confirmed that cells cultured on the PMGI aligned fiber sheet propagated in the direction of the aligned fibers. On the other hand, when the cells were cultured on the Flat, the cells tended to aggregate and weakly adhered to the Flat (Figure 5A). The cells were cultured for 14 days and then, the fiber sheet with the cultured cardiomyocytes was peeled off from the MEA with tweezers. The cells were firmly adhered on the fiber sheet and no cell or fiber remained on the MEA was observed (Figure 5B).

### C. Measurement of electrical signal from the cardiomyocytes over time

Electrical signals from the cardiomyocytes cultured on the PMGI aligned fiber sheet-coated (AF) and gelatin-coated (Flat) MEAs were measured on day 2, 6, 10 and 14 of culture (Figure 5C). The signal detection rate in the AF sample increased with the time of culture and on day 6 and thereafter, all channels detected signal (the percentage of the channels that detected signal was 100%). On the other hand, the percentage of the channels that detected signal on day 6 of culturing the cardiomyocytes on the Flat MEA was less than that of the cells cultured on AF MEA and was about 60-80%. After 14 days from the start of the culture, the cells on the Flat MEA tended to detach from the substrate, whereas the cells cultured on the PMGI aligned fiber sheet did not detach from the substrate (Figure 5D) and the signal detection rate did not decrease.

### 4-2. Action potential of cardiomyocytes cultured on the PLGA aligned fiber sheet

Similarly, cardiomyocytes induced from human iPS cells (IMR90-1) were seeded on the PLGA aligned fiber sheet-coated (AF, spin time: 40 seconds, thickness: 2 µm, fiber density: 300 fibers/mm), PLGA random fiber sheet-coated (RF, spin time: 20 seconds, thickness: 2 µm) and gelatin-coated (Flat) MEAs and cultured. The electrical signals from the cells were measured. Irrespective of the structure of the fiber sheet, the signal detection rate with the cells cultured on the fiber sheet-coated MEAs increased with the time of culture and on day 6 and thereafter, all channels detected signal (the percentage of the channels that detected signal was 100%). The signal detection rate did not decrease after 14 days of culture. The percentage of the channels that detected signal on day 6 of culturing the cardiomyocytes on the Flat MEA was less than that of the cells cultured on the fiber sheet-coated MEAs and was about 60-80% (Figure 5E). The cells on the Flat MEA tended to detach from the substrate after day 10 of culture whereas the cells cultured on the PMGI aligned fiber sheet-coated MEA did not detach from the substrate even on day 32 of culture (Figure 5F). Those observations suggest that the cell attachment rate were high when the cells were cultured on the fiber sheet coated-MEAs and therefore, the electrical signal detection rates were high. Whereas the cell attachment rate was low and the cellular density was ununiform when the cells were cultured on the gelatin-coated MEA and therefore, the signal detection rate was low.

The cell attachment rates between the cells on the PLGA aligned fiber sheet and the cells on the gelatin flat were similar at 5 hours from the seeding of the cell (Figure 3A). While after a long term culture of the cells, the cell attachment rate of the cells on the PLGA aligned fiber sheet was significantly higher than the cells on the gelatin coated substrate (Figure 5F). Those results confirm that the cells stably attach on the aligned fiber sheet for long time.

### EXAMPLE 5: Evaluation of functions of cardiomyocytes on the aligned fiber sheet

### 5-1. Action potential pattern of the cardiomyocytes on the PMGI aligned fiber sheet.

### Detection of arrhythmia

PMGI aligned fiber sheet-coated (AF, spin time: 90 seconds, thickness: 2 µm, fiber density: 300 fibers/mm) and gelatin-coated (Flat) MEAs prepared according to the procedures of Example 3 were used. Cardiomyocytes induced from human iPS cells (IMR90-1) were seeded on each MEA and cultured. The electrical signals from the cells were detected according to the procedures of Example 4 (n=4). No irregular electrical signal corresponding to arrhythmia was observed from the cells cultured on the PMGI aligned fiber sheet for 6 days from the start of the culture (Figure 6A upper and Figure 6B). Whereas, arrhythmia were detected with a probability of 40% in the cells cultured on the Flat-coated MEA with respect to the number of the experimental samples (Figure 6A upper and Figure 6B).

### Signal Amplitude

Higher signal amplitude was observed from the cardiomyocytes cultured on the PMGI aligned fiber sheet (AF) compared to those on the Flat (Figure 6A and Figure 6C). Further, the signal amplitudes of the cells at different culture times were recorded for 14 days. The signal amplitude of the cells cultured on the Flat-coated MEA did not change while that of the cells cultured on the aligned fiber sheet increased with time (Figure 6D).

### 5-2. Action potential pattern of the cardiomyocytes on the PLGA aligned fiber sheet.

A high density PLGA aligned fiber sheet-coated (AF-H, spin time: 10 minutes, thickness: 10 µm, fiber density: 10000 fibers/mm), low density PLGA aligned fiber sheet-coated (AF-L, spin time: 40 seconds, thickness: 2 µm, fiber density: 300 fibers/mm), PLGA random fiber sheet-coated (RF, spin time: 20 seconds, thickness: 2 µm) and gelatin-coated (Flat) MEAs were used. Cardiomyocytes induced from human iPS cells (IMR90-1) were cultured on each MEA and the electrical signals from the cells on day 6 of culture were measured. In addition, the signal amplitudes of the cells at different culture times were recorded for 14 day. Irrespective of the difference in the structures of the fiber sheets, higher signal amplitudes were observed from the cardiomyocytes cultured on the fiber sheet-coated MEAs compared to those on the Flat. In addition, no irregular electrical signal corresponding to arrhythmia was observed from the cells cultured on the fiber sheet-coated MEAs (Figure 6E). Whereas, arrhythmia were frequently observed in the cells cultured on the gelatin-coated MEA (Flat) . (Figures 6E and 6F) The arrhythmia detection rate was 34.5% in the cells cultured on the Flat-coated MEA with respect to the number of the experimental samples. Further, the signal amplitudes of the cells at different culture times were recorded for 14 days. The signal amplitude of the cells cultured on the Flat-coated MEA did not change while that of the cells cultured on the fiber sheet coated MEAs increased with time (Figure 6G). Especially, after day 10 of culture, significantly higher signal amplitudes were observed from the cardiomyocytes cultured on the aligned fiber sheet-coated MEAs compared to those on random fiber sheet coated-MEA.

According to those results, it was confirmed that the cardiomyocytes are preferably cultured on a fiber sheet mounted on the MEA to give stable culture of normal cells that unlikely incur arrhythmia. Those results are obtained by culturing the cardiomyocytes on the aligned fiber sheet mounted on MEAs because the cells preferably adhere to the MEA through the fibers and disperse uniformly on the MEA.

### EXAMPLE 6: Excitation propagation in a cardiac tissue-like construct on the aligned fiber sheet, direction and speed 6-1. Excitation propagation in a cardiac tissue-like construct on the PMGI aligned fiber sheet.

The direction and speed of cardiomyocyte excitation propagation were measured and evaluated. They are important for evaluating the cardiac functions. According to the procedures of Examples 3 and 4, cardiomyocytes induced from human iPS cells were cultured on the PMGI aligned fiber sheet-coated (AF, spin time: 90 seconds, thickness 2µm, fiber density: 300 fibers/mm) and gelatin-coated (Flat) MEAs. On day 14 of culture, electrical stimulation (±1500 mV, 40µs duration) was applied to the cultured cells at the middle part of the field of view and the electrical signals from the cells were measured. Activation maps showing the propagation of electrical excitation were prepared (Figure 7A). The change in the shape of the contour line represents the direction of propagation and the change in the color represents the propagation speed. The propagation speeds on day 14 of culture were quantitatively compared (Figure 7B).

The direction of the propagation of cardiomyocyte excitation was consistent with the direction of the aligned fibers. The propagation speed along the fiber direction was significantly larger than that perpendicular to the fiber direction or that of the cardiomyocytes cultured on the gelatin-coated MEA. On the other hand, when the cells were cultured on the gelatin-coated MEA, the start point of the excitation was distant from the point of stimulation (Figure 7A, right). This may be thought to be because the cells on the gelatin-coated substrate on day 14 of culture attach weakly to the substrate and tend to aggregate as shown by Figure 5D, and therefore, the cell distribution on the Flat MEA was ununiform. The excitation propagation speeds over time from the start to day 14 of culture were recorded. The propagation speed in the direction parallel to the aligned fibers after day 6 of culture was significantly higher compared with the cells cultured on the flat MEA (Figure 7C). Since PMGI is nonconductive, the aligned fiber sheet did not act as a conductive wire.

### 6-2. Excitation propagation in a cardiac tissue-like construct on the PLGA aligned fiber sheet.

According to the procedure of Example 6-1, cardiomyocytes induced from human iPS cells were cultured on the PLGA aligned fiber sheet-coated (AF, spin time: 40 seconds, thickness 2µm, fiber density: 300 fibers/mm), PLGA random fiber sheet-coated (RF, spin time: 20 seconds, thickness: 2 µm) and gelatin-coated (Flat) MEAs. On days 6 and 14 of culture, electrical stimulation (±1500 mV, 40µs duration) was applied to the cultured cells at the middle part of the field of view and the electrical signals from the cells were measured. Activation maps showing the propagation of electrical excitation were prepared (Figure 7D). The excitation propagation speeds on day 6 and 14 were quantified (Figure 7E) . Similar to the PMGI fiber sheet, the direction of the propagation of cardiomyocyte excitation was consistent with the direction of the PLGA aligned fibers (horizontal direction, Figure 7D, left). When there are no fibers, the start point of the excitation was distant from the point of stimulation (Figure 7D, right), similar to that observed in Example 6-1.

Excitation propagation on day 6 and day 14 of culture were compared. Irrespective of presence or absence, or the structure of the fibers, the excitation propagation speed became faster as the culture period was extended. On day 6 of culture, there was no difference in the propagation speed in the cardiomyocytes cultured on each substrate, while on day 14 of culture, it was confirmed that the propagation speed in the direction parallel to the direction of the PLGA aligned fiber sheet was about 3 times faster than that in the direction perpendicular to the aligned fibers, as well as than the propagation speed in the cells cultured on the flat substrate (Figure 7E). Since PLGA is nonconductive, the aligned fiber sheet did not act as a conductive wire.

Those results suggest that since the cardiomyocytes on the aligned fiber sheet have the aligned structure, the electrical coupling structure of the cells in the direction of the fibers developed so that the excitation propagation speed in the cultured cells in the direction parallel to the direction of the aligned fibers was significantly higher compared to the direction perpendicular to the aligned fibers or that of the cells cultured on the flat substrate. This means that the cardiomyocytes on the aligned fiber sheet had high maturity and functionality.

### EXAMPLE 7: Electrocardiogram of cardiomyocytes on the aligned fiber sheets

### 7-1. Electrocardiogram of cardiomyocytes on PMGI aligned fibers

In order to evaluate electrophysiological maturity of the cardiomyocytes and in a cardiac toxicity test using the cardiomyocytes, QT interval determined from the electrocardiogram can be used as a representative index. QT interval is the interval between the Q wave derived from the voltage-dependent Na⁺ current and the T wave derived from the voltage-dependent K⁺ current. According to the procedures of Examples 3 and 4, cardiomyocytes induced from human iPS cells (IMR90-1) were cultured on the PMGI aligned fiber sheet-coated (AF, spin time: 90 seconds, thickness 2µm, fiber density: 300 fibers/mm) and gelatin-coated (Flat) MEAs. On day 10 of culture, QT interval of each sample was measured. The cardiomyocytes on the PMGI aligned fiber sheet showed significantly shorter QT intervals than the cells on the gelatin-coated Flat MEA (Figure 8A). The QT intervals from the cardiomyocytes cultured under each condition were measured over time for 14 days. The QT intervals of the cardiomyocytes on the PMGI aligned fiber sheet was significantly shorter compared to that of the cells on the flat MEA. Those results indicate that the cardiomyocytes cultured on the aligned fiber sheet have a high electrophysiological maturity.

### 7-2. Electrocardiogram of cardiomyocytes on PLGA aligned fibers A. Measurement of T-wave

In addition to the QT intervals, the detection rate of the T-wave by electrocardiogram is also used as an index for evaluating electrophysiological maturity and cardiac toxicity of cardiomyocytes. According to the procedures of Examples 3 and 4, cardiomyocytes induced from human iPS cells (IMR90-1) were seeded on the low density PLGA aligned fiber sheet-coated (AF, spin time: 40 seconds, thickness: 2 µm, fiber density: 300 fibers/mm), the high density PLGA aligned fiber sheet-coated (AF-H, spin time: 10 minutes, thickness: 10 µm, fiber density: 10000 fibers/mm), the PLGA random fiber sheet-coated (RF, spin time: 20 seconds, thickness: 2 µm) and gelatin-coated (Flat) MEAs and cultured. The T-wave based on the voltage-dependent K⁺ current, which is corresponding to the T wave in the electrocardiogram was detected from the cultured cells over time using a MEA system for 14 days and the detection ratio was compared (Figure 8C).

After day 6 of culture, T-wave was detected from all channels from the cardiomyocytes cultured on the high density aligned fibers. On the other hand, the cardiomyocytes cultured on the low density aligned fibers/random fibers/gelatin coating showed low detection rates (50-80%) on day 6 of culture. Those results suggest that the maturities of the latter three cardiomyocytes were low. In addition, the T-wave detection ratio with the latter substrates further decreased with time. This result seems to be related to the weak adhesion between cells and electrodes. Further, when the Flat MEA was used, the detection rate of T-wave remarkably decreased after day 10 of culture. This is presumed to be due to the tendency of the cells to detach from the substrate (Figure. 5F)

### B. QT interval

The high density PLGA aligned fiber sheet-coated (AF-H, spin time: 10 minutes, thickness: 10 µm, fiber density: 10000 fibers/mm), the low density PLGA aligned fiber sheet-coated (AF, spin time: 40 seconds, thickness: 2 µm, fiber density: 300 fibers/mm), the PLGA random fiber sheet-coated (RF, spin time: 20 seconds, thickness: 2 µm) and gelatin-coated (Flat) MEAs were used. In the same manner as Example 7-1, cardiomyocytes were cultured on the substrates and QT intervals of the cells were measured on day 10 of culture. The QT interval of the cardiomyocytes on the aligned fiber sheet was shorter than the cardiomyocytes on the random fiber sheet and the Flat MEA. The cells cultured on the high density aligned fiber sheet (AF - H) showed further shortened QT interval (Figure 8D). Those results suggest that the cardiomyocytes cultured on the aligned fiber sheet have a high electrophysiological maturity, and that as the fiber density increased, the maturity increased.

In the same manner as Example 7-1, the QT intervals from the cardiomyocytes cultured under each condition were measured over time for 14 days (Figure 8E). The QT intervals did not change with ages of culture. The QT intervals of cardiomyocytes cultured on high density aligned fiber sheet (AF - H) were always the shortest. Those results suggest that that as the fiber density increased, the maturity increased. It was confirmed that on the low density aligned fiber sheet (AF) and the random fiber sheet (RF), the QT intervals were longer than the cardiomyocytes on the high density aligned fiber sheet, and the variations of the QT intervals among the samples were also large. Those results are considered to be related to the cell adhesion on the substrates. On the other hand, the longest QT intervals were observed with the cells on the Flat MEA, they were significantly longer than the QT intervals of the cardiomyocytes on the fiber sheet. Accordingly, it is considered that the electrophysiological maturity of the cardiomyocytes on the Flat MEA was low.

### 7-3. Electrocardiogram of cardiomyocytes on the PG aligned fibers

The PS aligned fiber sheet (spin time: 50 minutes, thickness: 10 µm, fiber density: 300 fibers/mm) was prepared according to Example 1-3 and used. The cardiomyocytes were cultured on the PS aligned fiber sheet-coated MEA and the QT interval of the cultured cells was measured on day 6 of culture. The QT interval of the cardiomyocytes on the PS aligned fiber sheet was very short as short as 0.16 seconds (Figure 8F). This value is close to the QT interval (about 0.2 seconds) observed in an adult human. This result means that the electrophysiological maturity of the cells on the aligned fiber sheet achieved to the same level as adult human.

### EXAMPLE 8: Immunostaining of the cardiac tissue-like construct using cardiomyocyte markers and RT-PCR

### 8-1. Immunostaining of the cardiac tissue-like construct produced on the PLGA aligned fiber sheet.

In order to determine maturity of the cells constructing the cardiac tissue-like construct prepared on the aligned fiber sheet, immunostaining with cardiac markers α-MHC, β-MHC, myosin light chain (MLC) -2V, cardiac troponin T (cTnT) 2 and α-Actinin were conducted. PLGA aligned fiber sheet-coated (spin time: 40 seconds, thickness: 2µm), PLGA random fiber sheet-coated (spin time: 20 seconds, thickness 2 µm) and gelatin-coated glass substrates were used. The cardiomyocytes derived from iPS cells were cultured on those substrates for 14 days. The cells were fixed with 4% formaldehyde solution for 15 minutes and washed three times with PBS for 5 minutes. Then, the cells were permeabilized in PBS plus 0.5% Triton X-100 for 30 minutes and washed three times with PBS for 5 minutes. After blocking in a 5% normal goat serum, 5% normal donkey serum, 3% BSA, and 0.1% Tween 20 in PBS for 1 hour, the cells were washed 3 times with PBS for 5 minutes. The cells were incubated with a primary antibody, an antibody against α-actinin (mouse monoclonal antibody EA-53; Sigma), cTnT2 (mouse monoclonal antibody: Santa Cruz Biotechnology, MLC2v (rabbit polyclonal antibody; Proteintech Group, USA), or β-MHC (mouse monoclonal antibody; Santa Cruz Biotechnology) at 4°C, overnight. The cells were then washed three times with PBS for 5 minutes. Then, the cells were incubated with different secondary antibodies: DyLight-594 anti-mouse IgG, DyLight 488 anti-mouse IgG, DyLight-594 anti-rabbit IgG, or DyLight-488 anti-mouse IgM at the room temperature for 1 h, and washed three times with PBS for 5 minutes. Nuclei of the cells were visualized by incubating the cells with 300nM 4,6-diamidino-2-phenylindole (DAPI; Invitrogen) at the room temperature for 30 minutes and washed three times with PBS. The cells were observed with a confocal laser scanning microscope (FV10i, Olympus).

Figure 9A represents DAPI signals that were merged with the fibers. As a result of observation, the expression of β-MHC, a marker relating the cardiac maturity in cardiomyocytes, was much higher in the cardiomyocytes on the fiber sheets, especially, on the aligned fiber sheet than that in the gelatin coating (figure 9A). In addition, the β-MHC RNA levels in the cardiomyocytes cultured on the aligned fiber sheet (AF) and on the gelatin coating (Flat) by means of RT-PCR. The β-MHC RNA level in the cardiomyocytes cultured on the gelatin coating was less than 10% of that in a normal cardiomyocytes derived from an adult. Whereas the β-MHC RNA level in the cardiomyocytes cultured on the aligned fiber sheet was increased to about 20% of that in a normal cardiomyocytes derived from an adult (Figure 9B). Those results suggest that the presence of the fibers, especially, the aligned fibers, the cardiomyocytes forms anisotropic 3D structure and the maturation of the cells are enhanced. In addition, from images of the cells stained with MLC2V, α-Actinin, and TnT2, the cardiomyocytes were aligned along the direction of the aligned fiber sheet. Further, the cTnT2 stained images were quantified by means of Fast Fourier Transformation and quantitatively confirmed that the direction of the cardiomyocytes was consistent with the direction of the aligned fibers (Figure 9C).

### 8-2. Immunostaining of the cardiac tissue-like construct produced on the PMGI aligned fiber sheet.

Similar to the above Example 8-1, cardiomyocytes induced from human iPS cells were cultured on PMGI aligned fiber sheet-coated (AF spin time: 90 seconds, thickness: 2µm) and gelatin-coated glass substrates respectively for 14 days. The obtained cells were subjected to immunostaining with primary antibodies to cardiac markers α-MHC and MLC2V, and to a cytoskelton marker actin (primary antibody, goat polyclonal antibody (Santa Cruz, SC 1616) and secondary antibody, Alexa Fluor 488 anti-goat IgG (Jackson ImmnoResearch #705-546-147) (Figure 9D). Similar to those observed with the PLGA aligned fiber sheet, the expression of β-MHC, a marker relating the cardiac maturity in cardiomyocytes, was much higher in the cardiomyocytes on the PMGI aligned fiber sheet than that in the gelatin coating. In addition, from images of the cells stained with MLC2V and Actin, the cardiomyocytes were aligned along the direction of the PMGI aligned fiber sheet. Those results suggest that the presence of the fibers, especially, the aligned fibers, the cardiomyocytes forms anisotropic 3D structure and the maturation of the cells are enhanced. In addition, from images of the cells stained with MLC2V and Actin, it was confirmed that the cardiomyocytes were aligned along the direction of the aligned fiber sheet.

### EXAMPLE 9. Electromicroscopic observation of the cardiac tissue-like construct on aligned fiber sheets.

### 9-1. Observation of cardiac tissue-like construct produced on the PLGA aligned fiber sheet.

In order to analyze the adherent between the fiber sheet and the cardiomyocytes and aligned structure of the cells and the 3D structure of the tissue-like construct, the tissue-like construct was observed with a transmission electron microscope (TEM). PLGA aligned fiber sheet-coated, PLGA random fiber sheet-coated, and gelatin-coated (Flat) plastic plates were prepared. According to the procedures in Example 2, cardiomyocytes induced from iPS cells were seeded on the plastic plates and cultured for 14 days. The cells were fixed with 2% glutaraldehyde solution in NaHCa buffer (100mM NaCl, 30mMHEPES, 2mM CaCl₂, pH7.4). Each sample was sequentially treated with 0.25% osmium, 0.25% K₄Fe (CN)₆, 1% tannic acid, and 50 mM uranyl acetate to perform late fixing treatment. Then, the samples were subjected to the ethanol series dehydration and then, to polymerization treatment at 65°C. Frozen sections were prepared in the direction perpendicular to the aligned fiber sheet so that the thickness was 60 to 100 nm, using an Ultra Microtome (Leica FC 6, Vienna, AU), and the cross section was observed with a transmission electron microscope (JEOL JEM 1400, Japan).

As a result of observing the section with the electron microscope, it was found that cardiomyocytes cells cultured on the PLGA fiber sheet were closely adhered so as to be wound around the fibers (FIG. 10A). Unlike the tissue-like construct prepared on the random fiber sheet, the muscle fibers in the tissue-like construct prepared on the aligned fiber sheet aligned in the direction coincided with the direction of the fibers. The thickness of the cardiac tissue-like construct prepared on the aligned fiber sheet was 30-50 µm. The tissue like construct had multiple cardiomyocyte layers generated on both sides of the fiber sheet and the number of the cell layers was about 10 (Figure 10 left).

### 9-2 Observation of cardiac tissue-like construct produced on the PMGI aligned fiber sheet.

Tissue-like constructs prepared by culturing cardiomyocytes on PMGI aligned fiber sheet-coated (spin time: 90 seconds, thickness: 2 µm) and gelatin coated plates were observed with a transmission electron microscope (TEM) in the same manner as in Example 9-1. Similar to the construct prepared on the PLGA aligned fiber sheet, cardiomyocytes cells cultured on the PMGI aligned fiber sheet were closely adhered so as to be wound around the fibers (FIG. 10B right). The thickness of the cardiac tissue-like construct prepared on the PMGI aligned fiber sheet was about 30 µm. The tissue like construct had multiple cardiomyocyte layers generated on both sides of the fiber sheet and the number of the cell layers was about 10 (Figure 10B, left).

### EXAMPLE 10 QT interval prolongation in response to an inhibitor of voltage-dependent potassium channel (HERG channel)

### A. QT interval prolongation in response to a HERG channel inhibitor, E-4031

According to the procedures of Examples 3 and 4, cardiomyocytes were cultured on the PMGI aligned fiber sheet-coated (AF, spin time: 40 seconds, thickness 2µm, fiber density: 300 fibers/mm), random fiber sheet-coated (RF, spin time 20 seconds, thickness 2 µm) and gelatin-coated (Flat) MEAs. A typical human Ether-a-go-go Related Gene (HERG) potassium ion channel inhibitor, E-4031 was applied to the cultured cells and the QT interval prolongation were sequentially observed for 5-10 minutes. The hERG potassium ion channel is an inward rectifier voltage-gated potassium channel in the heart. When the channel is blocked, QT interval prolongation will be observed. As the concentration of E-4031 increased or the maturity of the cultured cells lowered, arrhythmia may occur. Irrespective of the presence or absence of the fibers, or the structure of the fiber sheet, QT interval extension dependent on the concentration of E-4031 was observed (Figure 11A).

### B. Detection of arrhythmia after addition of the agent

Electrical signals from the cardiomyocytes were measured for 5-10 minutes from the application of E-4031. During the time period, the occurrence of arrhythmia was observed. As a result, arrhythmia was observed at a high frequency from cardiomyocytes cultured on flat MEA compared to the cells cultured on the fiber sheets (Figures 11B and 11C, and Table 1. Those results seem to be due to the low adhesion of the flat MEA to the cells and low maturity of the cells cultured on the flat substrate (Figure 9A).

**[Table 1]**

| sample | sample number in total | samples that occurred arrhythmia in response to E-4031 |
|---|---|---|
| Aligned fiber sheet | 9 | 1 |
| Random fiber sheet | 9 | 1 |
| Gelatin coating | 7 | 3 |

### EXAMPLE 11 Preparation of cardiac tissue-like construct with a sheet-shaped cell culture scaffold having a cell culture area and a frame around the cell culture area.

### A. Preparation of a sheet-shaped cell culture scaffold having a cell culture area made of an aligned fiber sheet.

According to the procedures of Example 1, PLGA aligned fiber sheet was prepared by means of electrospinning (spin time : 10 minutes, thickness: 10µm, fiber density: 10000 fibers/mm) and mounted on a spacer made from PDMS (1.8cm × 1.8cm). The same PLGA solution as that used for electrospinning the fibers was applied to the aligned fiber sheet to the area wherein the aligned fiber sheet and PDMS frame are contacted to give a frame. The sheet was left stood over night to dry and sterilized by UV radiation to give the sheet-shaped cell culture scaffold (figure 12A).

Sheet-shaped cell culture scaffolds with different materials for preparing the fibers and adhesives may be prepared in the same manner as above. Figure 12B depicts a sheet-shaped cell culture scaffold obtained by adhering the PLGA aligned fiber sheet (spin time: 10 minutes, thickness: 10 µm, fiber density: 10000 fibers/mm) to a PDMS spacer by the same PLGA solution as that used for electrospinning the PLGA fibers as adhesive. The frame can be prepared simultaneously with attaching the sheet to the spacer by applying a biodegradable adhesive to the spacer. Figure 12C depicts a sheet-shaped cell culture scaffold to which PDMS spacer is attached that was prepared by using a PS aligned fiber sheet (spin time: 50 minutes, thickness: 10 µm, fiber density: 300 fibers/mm) and a PDMS mixed solution (SLIPOT 184, prepolymer : crosslinking agent=10:1, Toray). Figure 12D depicts a sheet-shaped cell culture scaffold to which a glass ring is attached that was made of PLGA aligned fiber sheet (spin time:10 minutes, thickness: 10 µm, fiber density: 10000 fibers/mm) and glass ring having inner diameter of 22mm (Atzugi micro Co., Ltd). The aligned fiber sheet and the glass ring were attached by using one-component condensation cure type RTV silicone rubber (Shin-Etsu KE-45-T) . The glass ring may be used as a spacer. Alternatively, the culture medium may be introduced into the inside of the glass ring and the ring may be used as a cell culture chamber.

### B. Decomposition of biodegradable material, PLGA

For producing a tissue-tissue like construct to be used for implantation, the aligned fiber sheet is preferably prepared from a highly safe biodegradable material. PLGA that is a copolymer of poly lactic acid (PLA) and poly glycolic acid (PGA) has been known as non-toxic biodegradable material. The degradation speed can be controlled by adjusting the combination ratio of PLA and PGA. The degradation time of the aligned fiber sheet prepared in Example 11-A from PLGA (PLA75%:PGA25%) was examined under the cell culture condition. The cardiomyocytes were seeded and cultured according to the procedures of Example 2. After 3 months, the PLGA fiber sheet area (spin time: 10 minutes, thickness: 10µm, fiber density: 1000 fibers/mm) was degraded completely (Figure 12E).

### EXAMPLE 12 Implantation of the cardiac tissue-like construct to a heart.

### A. Attachment of cardiac tissue-like construct to a heart

Adhesion of tissue-like construct prepared on the aligned fiber sheet to a mouse excised heart was examined. A PLGA aligned fiber sheet (spin time: 10 minutes, thickness: 10 µm, fiber density: 1000 fibers/mm) having a PLGA frame prepared according to the procedures of Example 11-A was used. Cardiomyocytes were cultured by the same procedure of Example 2 on the sheet-shaped cell culture scaffold for 6 days to give tissue-like construct. The tissue-like construct containing the aligned fiber sheet was peeled off from the culture container and the construct was placed on the surface of a heart excised from a three months old mouse. After 3 hours, the tissue-like construct was attached to the pericardium (Figure 13 A). Even if the cardiac tissue-like construct was lifted with tweezers, it did not peel off from the mouse heart. Cardiac tissue-like construct was prepared similarly on a PLGA aligned fiber sheet having a PDMS frame area and the three sides of the frame were cut off. Only the cell culture area was placed on the surface of a mouse heart and the heart was left stood. Adherence of the cardiac tissue-like construct to the surface of the heart was also observed (Figure 13B).

### B. In vivo implantation of the cardiac tissue-like construct into a rat

A cardiac tissue like construct was produced in the same manner as in Example 12-A above on the PLGA aligned fiber sheet (spin time: 10 minutes, thickness: 10 µm, fiber density: 1000 fibers/mm) having a PLGA frame was used. On day 6 of culture of cardiomyocytes, the tissue like-construct was implanted on the surface of a nude-rat. The heart was removed from the body two weeks after the implantation, fixed and dehydrated with 4% paraformaldehyde, embedded with paraffin and cut into 2 µm slices. The slices were deparaffinized and stained by means of hematoxyline-eosin (HE), by means of in situ hybridization with a human-specific probe (ISH) and by means of cTnT immunostaining (cTnT), then, microscopically observed (Figure 3C). The implanted cardiac tissue-like construct was engrafted on the surface of the heart to which the construct was implanted and the cardiomyocytes induced from human iPS cells were observed on the surface of the heart of the host.

### EXAMPLE 13: Multi-layered cardiac tissue-like construct produced on the fibers

### A. Duplication of cardiac-tissue like constructs produced on the aligned fibers

We investigated whether it is possible to produce thicker cardiac tissue-like construct by stacking tissue-like constructs that were produced on the aligned fiber sheets. According to the procedures of Example 11, a sheet-like cell culture scaffold having cell culture area of PLGA aligned fiber sheet (spin time: 10 minutes, thickness: 10 µm) having a PLGA frame equipped with PDMS spacer. According to the procedures of Example, 2, the cardiomyocytes induced from iPS cells (IMR90-1) were seeded on the PLGA aligned fiber sheet and cultured. On day 14 of culture, two cardiac tissue-like constructs wherein cardiomyocytes were proliferated on both sides of the PLGA aligned fiber sheet were stacked so that the directions of the fibers were same (Figure 14A, upper). The stacked tissue-like constructs were adhered to each other after 2 hours. The two-tissue like constructs were adhered firmly and the two constructs could not be separated by using tweezers (Figure 14A, lower). Those results indicate that a multilayer construct can easily be produced from a plurality of the cardiac tissue-like constructs produced on the aligned fiber sheets.

### B. Beat of the two-layered cardiac tissue-like construct

In order to evaluate the functions of the multilayer cardiac tissue-like construct with PLGA aligned fiber sheets obtained in Example 12-A above, the obtained multi-layer tissue like construct was peeled off from the PDMS spacer and placed on a multi-electrode array (30 µm diameter, 200 µm spacing 8 × 8 grid array) and detected the electrical signals. At after one minute from stacking the two cardiac tissue-like constructs, each construct beat independently, while at after 3 hours, the two layers of cardiac tissue-like constructs beat synchronously (Figure 14B).

### REFERENCE SIGNS LIST

- 1:: Aligned fibers
- 2:: Multi-electrode array
- 3:: Aligned fiber sheet + Multi-electrode array
- 4:: Cell culture chamber
- 5:: Multi-electrode array
- 6:: Frame
- 7:: Spacer
- 8:: Cardiac tissue-like construct.

## Claims

1. A cardiac tissue-like construct comprising an aligned fiber sheet and cultured cardiomyocytes on the sheet.

2. The cardiac tissue-like construct according to claim 1, wherein the aligned fiber sheet is interposed between the cultured cardiomyocyte layers.

3. The cardiac tissue-like construct according to claim 1 or 2, wherein the cultured cardiomyocytes are those induced from pluripotent stem cells.

4. The cardiac tissue-like construct according to any one of claims 1 to 3, wherein the diameter of the fibers constituting the aligned fiber sheet is 0.1 µm-5 µm, the number of the fibers per 1mm width of the sheet is 30-15000, and the thickness of the sheet is 0.1 µm-20 µm.

5. The cardiac tissue-like construct according to any one of claims 1 to 4, which comprises 10 or more cardiomyocytes layers.

6. The cardiac tissue-like construct according to any one of claims 1 to 5, wherein the fibers constituting the aligned fiber sheet is made from a biodegradable material.

7. The cardiac tissue-like construct according to any one of claims 1 to 5, wherein the fibers constituting the aligned fiber sheet is made from a hardly degradable material.

8. The cardiac tissue-like construct according to any one of claims 1 to 7, wherein the aligned fiber sheet has a frame around the sheet.

9. The cardiac tissue-like construct according to claim 8, wherein the frame is made from the same material as the aligned fiber sheet.

10. The cardiac tissue-like construct according to claim 8, wherein the frame is made from a material other than the aligned fiber sheet.

11. The cardiac tissue-like construct according to any one of claims 1-10, which comprises a plurality of aligned fiber sheets and a plurality of cardiomyocyte layers cultured on the fiber sheets, and the aligned fiber sheets are interposed between cardiomyocyte layers so as to the fiber direction of the aligned fibers are same.

12. The cardiac tissue-like construct according to any one of claims 1-4, 6 and 7, further comprises a cell culture chamber on the aligned fiber sheet, and the cardiomyocytes are in the culture chamber.

13. The cardiac tissue-like construct according to any one of claims 1-12, wherein the expression level of β-MHC in the cardiomyocytes in the cardiac tissue-like construct is 10% or more of the normal cardiomyocytes in adult human.

14. The cardiac tissue-like construct according to any one of claims 1-13, which is for use in evaluating cell functions.

15. The cardiac tissue-like construct according to any one of claims 6, 8-11 and 13, which is for use in implantation.

16. A method for producing the cardiac tissue-like construct of claim 11, which comprises the step of stacking a plurality of cardiac tissue-like constructs so that the aligned fibers in each construct are in the same direction and culturing the stacked constructs.

17. The method according to claim 16, further comprising the step of evaluating the cardiac tissue-like construct based on electrical signals from the cultured cardiomyocytes detected by means of a multiple electrode array that is contacted with the cardiac tissue-like construct.

18. A method for evaluating the function of cardiomyocytes, which comprises the steps of contacting the cardiac tissue-like construct according to claim14 with a multiple electrode array and detecting the electrical signals from the cardiac tissue-like construct.

19. The method according to claim 18, which is for evaluating the effectiveness of a drug candidate substance based on the function of cardiomyocytes.

20. The method according to claim 18, which is for evaluating the safety of a substance based on the function of cardiomyocytes.

21. A product comprising a sterilized package and a cardiac tissue-like construct according to any one of claims 1-15 enclosed in the package.

22. A device for evaluating function of cardiomyocytes, comprising, a multiple electrode array, and an aligned fiber sheet on the multiple electrode array.

23. The device according to claim 22, further comprises a cell culture chamber moiety on the aligned fiber sheet.

24. The device according to claim 23, further comprises cultured cardiomyocytes in the chamber moiety.

25. A method for evaluating cardiomyocyte function, which comprises the step of detecting the electrical signal from the cultured cardiomyocytes in the device of 24.

26. A sheet-shaped cell culture scaffold, comprising a sheet-shaped cell culture area and a frame provided around the cell culture area.

27. The sheet-shaped cell culture scaffold according to claim 26, wherein the sheet-shaped cell culture area is made of an aligned fiber sheet.

28. The sheet-shaped cell culture scaffold according to claim 26 or 27, wherein the sheet-shaped cell culture area and the frame are made from same material.

29. The sheet-shaped cell culture scaffold according to claim 26 or 27, wherein the sheet-shaped cell culture area and the frame are made from different materials.

30. The sheet-shaped cell culture scaffold according to any one of claims 27-29, wherein the sheet-shaped cell culture area is made of a randomly integrated fiber sheet.

31. The sheet-shaped cell culture scaffold according to any one of claims 27-29, wherein the sheet-shaped cell culture area is made of an aligned fiber sheet.

32. The sheet-shaped cell culture scaffold according to any one of claims 27-31, wherein the sheet-shaped cell culture area is made from a biodegradable material.

33. The sheet-shaped cell culture scaffold according to any one of claims 27-31, wherein the sheet-shaped cell culture area is made from a hardly degradable material.

34. The sheet-shaped cell culture scaffold according to any one of claims 26-33, which has a spacer on the frame.

35. A tissue-like construct comprising: the sheet-shaped cell culture scaffold according to any one of claims 26-34 and cultured cells on the scaffold.

36. The tissue-like construct according to claim 35, wherein a plurality of sheet-shaped cell culture scaffolds are interposed between cultured cell layers.

37. A product comprising, a sterilized package, and a tissue-like construct according to claim 35 or 36 enclosed in the package.
